# EUROPEAN PATENT APPLICATION

(11) **EP 4 410 841 A1**
(43) Date of publication of application: **07.08.2024**
(21) Application number: 22875179.8
(22) Date of filing: 30.09.2022
(51) Int. Cl.: C07K 16/46, C07K 16/24, C07K 19/00, C12N 15/62, A61K 39/395, A61P 37/02

(54) **ANTI-IL23 ANTIBODY FUSION PROTEIN AND USES THEREOF**

(30) Priority: 30.09.2021 CN 202111161893
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: MAO, Langyong, Shanghai 200245 (CN); YING, Hua, Shanghai 200245 (CN); XUE, Yi, Shanghai 200245 (CN); JIN, Xinsheng, Shanghai 200245 (CN); TAO, Weikang, Shanghai 200245 (CN)
(74) Representative: Potter Clarkson
(86) International application number: PCT/CN2022/123370
(87) International publication number: WO 2023/051798

(57) **Abstract**

An anti-IL23 antibody fusion protein and uses thereof, the anti-IL23 antibody fusion protein containing anti IL23 antibodies and TAC polypeptides, and the IL23 antibodies specifically binding the human IL23 p19 subunit. The anti-IL23 antibody fusion protein can be used for the treatment or improvement of B cell disorders or autoimmune diseases.

## Description

### TECHNICAL FIELD

The present disclosure belongs to the field of biotechnology, and particularly, the present disclosure relates to an anti-IL23 antibody, a fusion protein thereof, and use thereof.

### BACKGROUND

The statements herein merely provide background information related to the present disclosure and may not necessarily constitute the prior art.

IL-23 (also known as IL23) is mainly produced by activated dendritic cells, macrophages, monocytes, etc. It is a member of the IL-12 heterodimer cytokine family and consists of two subunits, p19 (also known as IL23 p19 subunit) and p40 (also known as IL23 p40 subunit), wherein the p40 subunit is a subunit also contained in IL-12 (J Immunol. 2018 Sep 15; 201(6): 1605-1613).

IL-23 exerts its biological functions by interacting with its receptors and activating downstream signaling pathways. IL-23 receptors include the IL-12 receptor β1 and 2 subunits of the IL-23 receptor (J Immunol. 2002 Jun 1;168(11): 5699-708). IL-23 can promote the differentiation of Th17 cells, plays an important role in the proliferation and stabilization of Th17 cells, and can promote Th17 cells to produce cytokines such as IL-17A, IL-17F, and IL-22, and the inflammatory factors act on keratinocytes, resulting in activation and over-proliferation of the keratinocytes. Activated keratinocytes recruit and activate immune cells such as T cells by producing a large number of cytokines, chemokines, antimicrobial peptides, etc., and form a cascade effect of immune responses, causing the symptoms of psoriasis (Cutis. 2018 Mar;101(3S): 5-9).

Systemic lupus erythematosus(SLE) is an autoimmune disease caused by over-activation of the immune response (Nat Rev Dis Primers. 2016 Jun 16; 2: 16039). A variety of cells are involved in the progression of SLE. The beginning is related to the neutrophils and apoptotic cells. The neutrophils release a reticular extracellular trap to capture antibacterial protein, and pDC is stimulated to secrete a large amount of type I interferon under the combined action of interferon. The apoptotic cells may release high-mobility proteins and nucleic acids in the nucleus to act as the antigen of themselves to induce immune cells to generate the nucleotide antibody, and the immune complex of the nucleic acid and the antibody interacts with the receptor on the cell surface to activate TLR signals, and pDC is stimulated to secrete interferon. Interferon further activates mDC to produce various cytokines that activate T cells and B cells (Curr Opin Rheumatol. 2017 Mar; 29(2): 178-186). IL-12 and IL-23 are two cytokines known to be capable of activating T cell differentiation. After T cells are activated, various cytokines are secreted. Cytokines like IL-17 are involved in recruitment of inflammatory cells and cause tissue damage, and cytokines like IL-21 are involved in the co-stimulation of B cells. BAFF and APRIL are B cell activators, which stimulate B cells to differentiate, mature, and secrete antibodies through interaction with receptors on the surface of B cells (Nat Rev Rheumatol. 2016 Nov 22; 12(12): 716-730).

TACI is a membrane-bound receptor with an extracellular region comprising two cysteine-rich pseudo-repeats, a transmembrane region, and a cytoplasmic region that interacts with CAML (calcium-modulating cyclophilin ligand). TACI is associated with one subtype of B cells and T cells. TACI receptors bind to BAFF of the tumor necrosis factor ligand family. BAFF is a B cell activator in TNF family. It is mainly expressed on the surface of a bone marrow cell membrane and exists in the form of a trimer. The BAFF on the surface of the cell membrane can be hydrolyzed by protease to form soluble BAFF that enters the blood circulation system, and the soluble BAFF has the characteristic of polymerization and can form a 60-mer at most. In addition, BAFF can also interact with another protein of the same family, APRIL, to form a heterotrimer. Three receptors for BAFF, i.e., BAFF-R, BCMA, and TACI, are currently known on the surface of B cells. BAFF interacts with these three receptors and is involved in the differentiation maturation, survival, and regulation of B cells. APRIL shares two receptors with BAFF, i.e., BCMA and TACI, and functions with these two receptors to participate in the survival and regulation of B cells (Samy, E., et al., Int Rev Immunol, 2017. 36: p. 3-19; Kamal, A. and M. Khamashta, Autoimmun Rev, 2014. 13: p. 1094-1101). BAFF is important for maintaining B cell homeostasis, and over-activation of the BAFF signaling pathway leads to the survival of autoreactive B cells and the production of autoantibodies that promote autoimmune responses (Cancro, M.P., D.P. D'Cruz, and M.A. Khamashta, J Clin Invest, 2009. 119: p. 1066-73).

### SUMMARY

The present disclosure constructs an anti-IL23 antibody fusion protein comprising an anti-IL23 antibody and a TACI polypeptide, wherein the anti-IL23 antibody specifically binds to human IL23 p19 subunit.

According to some embodiments of the anti-IL23 antibody fusion protein of the present disclosure, the anti-IL23 antibody fusion protein comprises an anti-IL23 antibody fused to a TACI polypeptide or consists of the anti-IL23 antibody and the TACI polypeptide; preferably, the TACI polypeptide is fused to the anti-IL23 antibody at the N-segment and/or C-terminus of the heavy chain of the anti-IL23 antibody.

In some embodiments, the anti-IL23 antibody in the anti-IL23 antibody fusion protein comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein
(i) the HCDR1, the HCDR2, and the HCDR3 of the heavy chain variable region comprise amino acid sequences of a HCDR1, a HCDR2, and a HCDR3, respectively, in SEQ ID NO: 22, 21, 20, 19, 18, 17, or 1, and the LCDR1, the LCDR2, and the LCDR3 of the light chain variable region comprise amino acid sequences of a LCDR1, a LCDR2, and a LCDR3, respectively, in SEQ ID NO: 26, 25, 24, 23, or 2; or
(ii) the HCDR1, the HCDR2, and the HCDR3 of the heavy chain variable region comprise amino acid sequences of a HCDR1, a HCDR2, and a HCDR3, respectively, in SEQ ID NO: 29, 30, 31, 32, or 3, and the LCDR1, the LCDR2, and the LCDR3 of the light chain variable region comprise amino acid sequences of a LCDR1, a LCDR2, and a LCDR3, respectively, in SEQ ID NO: 36, 35, 34, 33, or 4.

In some embodiments of the anti-IL23 antibody fusion protein according to any one of the above, in the anti-IL23 antibody, the HCDR1, the HCDR2, and the HCDR3 of the heavy chain variable region and the LCDR1, the LCDR2, and the LCDR3 of the light chain variable region are defined according to the numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme; in some embodiments, the HCDR1, the HCDR2, and the HCDR3 of the heavy chain variable region and the LCDR1, the LCDR2, and the LCDR3 of the light chain variable region are defined according to the IMGT numbering scheme; in some embodiments, the HCDR1, the HCDR2, and the HCDR3 of the heavy chain variable region and the LCDR1, the LCDR2, and the LCDR3 of the light chain variable region are defined according to the Chothia numbering scheme; in some embodiments, the HCDR1, the HCDR2, and the HCDR3 of the heavy chain variable region and the LCDR1, the LCDR2, and the LCDR3 of the light chain variable region are defined according to the AbM numbering scheme; in some embodiments, the HCDR1, the HCDR2, and the HCDR3 of the heavy chain variable region and the LCDR1, the LCDR2, and the LCDR3 of the light chain variable region are defined according to the Contact numbering scheme.

In some embodiments of the anti-IL23 antibody fusion protein according to any one of the above, the anti-IL23 antibody comprises a heavy chain variable region and a light chain variable region, wherein
(i) in the heavy chain variable region, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(ii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16.

In some embodiments of the anti-IL23 antibody fusion protein according to any one of the above, the anti-IL23 antibody comprises a heavy chain variable region and a light chain variable region, wherein
(i) in the heavy chain variable region, the HCDR1 comprises the amino acid sequence of SEQ ID NO: 27, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(ii) in the heavy chain variable region, the HCDR1 comprises the amino acid sequence of SEQ ID NO: 28, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(iii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37, and the LCDR3 comprises the amino acid sequence of SEQ ID NO: 16.

In some embodiments of the anti-IL23 antibody fusion protein according to any one of the above, the anti-IL23 antibody is a murine antibody, a chimeric antibody, or a humanized antibody. In some embodiments, the antibody is a humanized antibody.

In some embodiments of the anti-IL23 antibody fusion protein according to any one of the above, the anti-IL23 antibody comprises a heavy chain variable region and a light chain variable region, wherein
(i) the heavy chain variable region comprises any one of SEQ ID NO: 22, 21, 20, 19, 18, or 17, or an amino acid sequence having at least 90% (e.g., at least 90%, 95%, 96%, 97%, 98%, or 99%) sequence identity thereto, and the light chain variable region comprises any one of SEQ ID NO: 26, 25, 24, or 23, or an amino acid sequence having at least 90% (e.g., at least 90%, 95%, 96%, 97%, 98%, or 99%) sequence identity thereto; or
(ii) the heavy chain variable region comprises any one of SEQ ID NO: 29, 30, 31, or 32 or an amino acid sequence having at least 90% (e.g., at least 90%, 95%, 96%, 97%, 98%, or 99%) sequence identity thereto, and the light chain variable region comprises any one of SEQ ID NO: 36, 35, 34, or 33, or an amino acid sequence having at least 90% (e.g., at least 90%, 95%, 96%, 97%, 98%, or 99%) sequence identity thereto; or
(iii) the heavy chain variable region comprises SEQ ID NO: 1 or an amino acid sequence having at least 90% (e.g., at least 90%, 95%, 96%, 97%, 98%, or 99%) sequence identity thereto, and the light chain variable region comprises SEQ ID NO: 2 or an amino acid sequence having at least 90% (e.g., at least 90%, 95%, 96%, 97%, 98%, or 99%) sequence identity thereto; or
(iv) the heavy chain variable region comprises SEQ ID NO: 3 or an amino acid sequence having at least 90% (e.g., at least 90%, 95%, 96%, 97%, 98%, or 99%) sequence identity thereto, and the light chain variable region comprises SEQ ID NO: 4 or an amino acid sequence having at least 90% (e.g., at least 90%, 95%, 96%, 97%, 98%, or 99%) sequence identity thereto.

In some embodiments of the anti-IL23 antibody fusion protein according to any one of the above, in the anti-IL23 antibody, the heavy chain variable region comprises, in a framework region thereof, one or more amino acid mutations selected from the group consisting of positions 1, 30, 37, 44, 49, 73, 89, and 93 (numbering according to Kabat numbering scheme), and/or the light chain variable region comprises, in a framework region thereof, one or more amino acid mutations selected from the group consisting of positions 4, 17, 36, 58, 60, and 68 (numbering according to Kabat numbering scheme). In some embodiments of the anti-IL23 antibody fusion protein, in the heavy chain variable region of the anti-IL23 antibody, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; in the antibody, the heavy chain variable region comprises, in a framework region thereof, one or more amino acid mutations selected from the group consisting of 1E, 30T, 37V, 44G, 49G, 73N, 89R, and 93V (numbering according to Kabat numbering scheme), and/or the light chain variable region comprises, in a framework region thereof, one or more amino acid mutations selected from the group consisting of 4L, 17Q, 36F, 58I, 60A, and 68R (numbering according to Kabat numbering scheme).

In some embodiments of the anti-IL23 antibody fusion protein according to any one of the above, in the anti-IL23 antibody, the heavy chain variable region comprises, in a framework region thereof, one or more amino acid mutations selected from the group consisting of positions 1, 2, 28, 44, 71, and 73 (numbering according to Kabat numbering scheme), and/or the light chain variable region comprises, in a framework region thereof, one or more amino acid mutations selected from the group consisting of positions 4, 36, 39, 71, and 100 (numbering according to Kabat numbering scheme). In some embodiments of the anti-IL23 antibody fusion protein, in the heavy chain variable region of the anti-IL23 antibody, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16; in the antibody, the heavy chain variable region comprises, in a framework region thereof, one or more amino acid mutations selected from the group consisting of 1E, 2F, 28S, 44S, 71V, and 73Q (numbering according to Kabat numbering scheme), and/or the light chain variable region comprises, in a framework region thereof, one or more amino acid mutations selected from the group consisting of 4I, 36F, 39R, 71L, and 100S (numbering according to Kabat numbering scheme).

In some embodiments of the anti-IL23 antibody fusion protein according to any one of the above, the anti-IL23 antibody comprises a heavy chain variable region and a light chain variable region, wherein
(i) the heavy chain variable region comprises the amino acid sequence of SEQ ID NO: 22, 21, 20, 19, 18, or 17, and the light chain variable region comprises the amino acid sequence of SEQ ID NO: 26, 25, 24, or 23; or
(ii) the heavy chain variable region comprises the amino acid sequence of SEQ ID NO: 29, 30, 31, or 32, and the light chain variable region comprises the amino acid sequence of SEQ ID NO: 36, 35, 34, or 33; or
(iii) the heavy chain variable region comprises the amino acid sequence of SEQ ID NO: 1, and the light chain variable region comprises the amino acid sequence of SEQ ID NO: 2; or
(iv) the heavy chain variable region comprises the amino acid sequence of SEQ ID NO: 3, and the light chain variable region comprises the amino acid sequence of SEQ ID NO: 4.

In some embodiments of the anti-IL23 antibody fusion protein according to any one of the above, the anti-IL23 antibody comprises a heavy chain variable region and a light chain variable region, wherein
(i) the heavy chain variable region comprises the amino acid sequence of SEQ ID NO: 22, and the light chain variable region comprises the amino acid sequence of SEQ ID NO: 26;
(ii) the heavy chain variable region comprises the amino acid sequence of SEQ ID NO: 21, and the light chain variable region comprises the amino acid sequence of SEQ ID NO: 25; or
(iii) the heavy chain variable region comprises the amino acid sequence of SEQ ID NO: 29, and the light chain variable region comprises the amino acid sequence of SEQ ID NO: 36.

In some embodiments of the anti-IL23 antibody fusion protein according to any one of the above, the anti-IL23 antibody further comprises antibody heavy and light chain constant regions. In some embodiments, the heavy chain constant region is a human IgG heavy chain constant region; in some embodiments, the heavy chain constant region is selected from the group consisting of human IgG1, IgG2, IgG3, and IgG4 constant regions; in some embodiments, the light chain constant region is selected from the group consisting of human antibody κ and λ chain constant regions; in some embodiments, the heavy chain constant region is a human IgG1 heavy chain constant region, and the light chain constant region is a human κ light chain constant region. In some embodiments, the Fc region of the heavy chain constant region has one or more amino acid substitutions capable of reducing the binding of the Fc region to an Fc receptor. In some embodiments, the Fc region has L234A and L235A mutations, and/or S228P mutation, and/or YTE mutations (M252Y, S254T, and T256E), and the numbering of the mutations is according to the EU index. In some embodiments, the heavy chain constant region comprises the amino acid sequence of SEQ ID NO: 38, and the light chain constant region comprises the amino acid sequence of SEQ ID NO: 39.

In some embodiments of the anti-IL23 antibody fusion protein according to any one of the above, the anti-IL23 antibody comprises a heavy chain and a light chain, wherein
(i) in the anti-IL23 antibody, the heavy chain comprises an amino acid sequence having at least 90% (e.g., at least 90%, 95%, 96%, 97%, 98%, or 99%) sequence identity to SEQ ID NO: 42, and the light chain comprises an amino acid sequence having at least 90% (e.g., at least 90%, 95%, 96%, 97%, 98%, or 99%) sequence identity to SEQ ID NO: 43; or
(ii) in the anti-IL23 antibody, the heavy chain comprises an amino acid sequence having at least 90% (e.g., at least 90%, 95%, 96%, 97%, 98%, or 99%) sequence identity to SEQ ID NO: 40, and the light chain comprises an amino acid sequence having at least 90% (e.g., at least 90%, 95%, 96%, 97%, 98%, or 99%) sequence identity to SEQ ID NO: 41; or
(iii) in the anti-IL23 antibody, the heavy chain comprises an amino acid sequence having at least 90% (e.g., at least 90%, 95%, 96%, 97%, 98%, or 99%) sequence identity to SEQ ID NO: 44, and the light chain comprises an amino acid sequence having at least 90% (e.g., at least 90%, 95%, 96%, 97%, 98%, or 99%) sequence identity to SEQ ID NO: 45.

In some embodiments of the anti-IL23 antibody fusion protein according to any one of the above, the anti-IL23 antibody comprises a heavy chain and a light chain, wherein
(i) in the anti-IL23 antibody, the heavy chain comprises the amino acid sequence of SEQ ID NO: 42, and the light chain comprises the amino acid sequence of SEQ ID NO: 43; or
(ii) in the anti-IL23 antibody, the heavy chain comprises the amino acid sequence of SEQ ID NO: 40, and the light chain comprises the amino acid sequence of SEQ ID NO: 41; or
(iii) in the anti-IL23 antibody, the heavy chain comprises the amino acid sequence of SEQ ID NO: 44, and the light chain comprises the amino acid sequence of SEQ ID NO: 45.

In some embodiments of the anti-IL23 antibody fusion protein according to any one of the above, the TACI polypeptide has a better function of preventing breaking.

In some embodiments of the anti-IL23 antibody fusion protein according to any one of the above, the TACI polypeptide is a polypeptide comprising amino acid residues at positions 48-85 of SEQ ID NO: 58 or a variant thereof, wherein the variant has an amino acid replacement at one or more positions selected from the group consisting of positions 49, 52, 53, 57, 65, 82, and 83, wherein the position for the amino acid replacement is an amino acid residue position numbered in natural order relative to the sequence SEQ ID NO: 58. In some embodiments, the variant of the TACI polypeptide has one or more amino acid replacements selected from the group consisting of 49T or 49R, 52S, 53E or 53Q, 57E, 65T or 65A, 82A or 82R, and 83Y, wherein the position for the amino acid replacement is an amino acid residue position numbered in natural order relative to the sequence SEQ ID NO: 58.

In some embodiments of the anti-IL23 antibody fusion protein according to any one of the above, the TACI polypeptide is set forth in SEQ ID NO: 58 or a truncated fragment of SEQ ID NO: 58 or a variant thereof, wherein the truncated fragment comprises amino acid residues at positions 48-85 of SEQ ID NO: 58, and the variant is a variant having one or more amino acid replacements at positions selected from the group consisting of positions 49, 52, 53, 57, 65, 82, and 83 in SEQ ID NO: 58 or the truncated fragment thereof, wherein the position for the amino acid replacement is an amino acid residue position numbered in natural order relative to the sequence SEQ ID NO: 58.

In some embodiments of the anti-IL23 antibody fusion protein according to any one of the above, the truncated fragment of the TACI polypeptide comprises amino acid residues at positions 48-86 of SEQ ID NO: 58, amino acid residues at positions 48-87 of SEQ ID NO: 58, or amino acid residues at positions 48-88 of SEQ ID NO: 58.

In some embodiments of the anti-IL23 antibody fusion protein according to any one of the above, the TACI polypeptide has the sequence set forth in any one of SEQ ID NOs: 60-63.

In some embodiments of the anti-IL23 antibody fusion protein according to any one of the above, the sequence of the TACI polypeptide is a variant of SEQ ID NO: 58 or a truncated fragment (e.g., a truncated fragment having the sequence of SEQ ID NO: 60, SEQ ID NO: 61, SEQ ID NO: 62, or SEQ ID NO: 63) of SEQ ID NO: 58, wherein the variant is a variant having any 1, 2, 3, 4, 5, 6, or 7 amino acid replacements at positions selected from the group consisting of positions 49, 52, 53, 57, 65, 82, and 83 in the sequence of SEQ ID NO: 58 or the truncated fragment thereof, wherein the position for the amino acid replacement is an amino acid residue position numbered in natural order relative to the sequence SEQ ID NO: 58.

In some embodiments of the anti-IL23 antibody fusion protein according to any one of the above, the TACI polypeptide is one having one or more amino acid replacements (e.g., 1, 2, 3, 4, 5, 6, or 7 amino acid replacements) selected from the group consisting of 49T or 49R, 52S, 53E or 53Q, 57E, 65T or 65A, 82A or 82R, and 83Y on the basis of the sequence of SEQ ID NO: 58, SEQ ID NO: 60, SEQ ID NO: 61, SEQ ID NO: 62, or SEQ ID NO: 63, wherein the position for the amino acid replacement is an amino acid residue position numbered in natural order relative to the sequence SEQ ID NO: 58.

In some embodiments of the anti-IL23 antibody fusion protein according to any one of the above, the TACI polypeptide is one having: any amino acid replacement selected from the group consisting of 49T, 52S, 53E, 53Q, 57E, and 82A on the basis of the sequence of SEQ ID NO: 58, SEQ ID NO: 60, SEQ ID NO: 61, SEQ ID NO: 62, or SEQ ID NO: 63; amino acid replacements 49R and 65T on the basis of the sequence of SEQ ID NO: 58, SEQ ID NO: 60, SEQ ID NO: 61, SEQ ID NO: 62, or SEQ ID NO: 63; amino acid replacements 45R and 65A on the basis of the sequence of SEQ ID NO: 58, SEQ ID NO: 60, SEQ ID NO: 61, SEQ ID NO: 62, or SEQ ID NO: 63; amino acid replacements 49R, 65T, and 82R on the basis of the sequence of SEQ ID NO: 58, SEQ ID NO: 60, SEQ ID NO: 61, SEQ ID NO: 62, or SEQ ID NO: 63; amino acid replacements 53E and 57E on the basis of the sequence of SEQ ID NO: 58, SEQ ID NO: 60, SEQ ID NO: 61, SEQ ID NO: 62, or SEQ ID NO: 63; amino acid replacements 52S, 53E, and 57E on the basis of the sequence of SEQ ID NO: 58, SEQ ID NO: 60, SEQ ID NO: 61, SEQ ID NO: 62, or SEQ ID NO: 63; amino acid replacements 49T and 82A on the basis of the sequence of SEQ ID NO: 58, SEQ ID NO: 60, SEQ ID NO: 61, SEQ ID NO: 62, or SEQ ID NO: 63; amino acid replacements 49T and 83Y on the basis of the sequence of SEQ ID NO: 58, SEQ ID NO: 60, SEQ ID NO: 61, SEQ ID NO: 62, or SEQ ID NO: 63; amino acid replacements 49T, 82A, and 83Y on the basis of the sequence of SEQ ID NO: 58, SEQ ID NO: 60, SEQ ID NO: 61, SEQ ID NO: 62, or SEQ ID NO: 63; or amino acid replacements 49T, 53E, 57E, and 82A on the basis of the sequence of SEQ ID NO: 58, SEQ ID NO: 60, SEQ ID NO: 61, SEQ ID NO: 62, or SEQ ID NO: 63. The positions for the amino acid replacements described above are amino acid residue positions numbered in natural order relative to the sequence SEQ ID NO: 58.

In some embodiments of the anti-IL23 antibody fusion protein according to any one of the above, the amino acid sequence of the TACI polypeptide is set forth in any one of SEQ ID NOs: 51-83.

In some embodiments of the anti-IL23 antibody fusion protein according to any one of the above, the amino acid sequence of the TACI polypeptide is set forth in any one of SEQ ID NOs: 60-63 and SEQ ID NOs: 66-83.

In some embodiments of the anti-IL23 antibody fusion protein according to any one of the above, the amino acid sequence of the TACI polypeptide is set forth in SEQ ID NO: 83.

In some embodiments, the anti-IL23 antibody fusion protein according to any one of the above comprises:
a first chain: [a TACI polypeptide 1]-[a linker 1]-[the heavy chain of the anti-IL23 antibody]-[a linker 2]-[a TACI polypeptide 2], and
a second chain: the light chain of the anti-IL23 antibody, wherein
the TACI polypeptide 1 and the TACI polypeptide 2 are identical or different; optionally, the TACI polypeptide 1 and the TACI polypeptide 2 may be independently selected from any TACI polypeptide described herein; the linker 1 and the linker 2 are identical or different; in some embodiments, the linker is a linker selected from the group consisting of (GₓS)_{y}, wherein x is selected from the group consisting of integers from 1 to 5 and y is selected from the group consisting of integers from 0 to 6; in some embodiments, the linker is a linker selected from the group consisting of (GₓS)_{y}, wherein x is selected from the group consisting of integers from 1 to 5 and y is selected from the group consisting of integers from 1 to 6; in some embodiments, the linker is GGGGSGGGGSGGGGS (set forth in SEQ ID NO: 84) or GGGS (set forth in SEQ ID NO: 85).

In some embodiments of the anti-IL23 antibody fusion protein according to any one of the above, the anti-IL23 antibody fusion protein has a first chain comprising the amino acid sequence of SEQ ID NO: 47 and a second chain comprising the amino acid sequence of SEQ ID NO: 43; or the anti-IL23 antibody fusion protein has a first chain comprising the amino acid sequence of SEQ ID NO: 46 and a second chain comprising the amino acid sequence of SEQ ID NO: 41. In some embodiments, the anti-IL23 antibody fusion protein has two first chains comprising the amino acid sequence of SEQ ID NO: 47 and two second chains comprising the amino acid sequence of SEQ ID NO: 43; in some embodiments, the anti-IL23 antibody fusion protein has two first chains comprising the amino acid sequence of SEQ ID NO: 46 and two second chains comprising the amino acid sequence of SEQ ID NO: 41.

In another aspect, the present disclosure provides an anti-IL23 antibody, wherein the anti-IL23 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein (i) the HCDR1, the HCDR2, and the HCDR3 of the heavy chain variable region comprise amino acid sequences of a HCDR1, a HCDR2, and a HCDR3, respectively, in SEQ ID NO: 22, 21, 20, 19, 18, 17, or 1, and the LCDR1, the LCDR2, and the LCDR3 of the light chain variable region comprise amino acid sequences of a LCDR1, a LCDR2, and a LCDR3, respectively, in SEQ ID NO: 26, 25, 24, 23, or 2; or (ii) the HCDR1, the HCDR2, and the HCDR3 of the heavy chain variable region comprise amino acid sequences of a HCDR1, a HCDR2, and a HCDR3, respectively, in SEQ ID NO: 29, 30, 31, 32, or 3, and the LCDR1, the LCDR2, and the LCDR3 of the light chain variable region comprise amino acid sequences of a LCDR1, a LCDR2, and a LCDR3, respectively, in SEQ ID NO: 36, 35, 34, 33, or 4.

In some embodiments of the anti-IL23 antibody according to any one of the above, the HCDR1, the HCDR2, and the HCDR3 of the heavy chain variable region and the LCDR1, the LCDR2, and the LCDR3 of the light chain variable region are defined according to the numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme; in some embodiments, the HCDR1, the HCDR2, and the HCDR3 of the heavy chain variable region and the LCDR1, the LCDR2, and the LCDR3 of the light chain variable region are defined according to the IMGT numbering scheme; in some embodiments, the HCDR1, the HCDR2, and the HCDR3 of the heavy chain variable region and the LCDR1, the LCDR2, and the LCDR3 of the light chain variable region are defined according to the Chothia numbering scheme; in some embodiments, the HCDR1, the HCDR2, and the HCDR3 of the heavy chain variable region and the LCDR1, the LCDR2, and the LCDR3 of the light chain variable region are defined according to the AbM numbering scheme; in some embodiments, the HCDR1, the HCDR2, and the HCDR3 of the heavy chain variable region and the LCDR1, the LCDR2, and the LCDR3 of the light chain variable region are defined according to the Contact numbering scheme.

In some embodiments of the anti-IL23 antibody according to any one of the above:
(i) in the heavy chain variable region, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(ii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16.

In some embodiments of the anti-IL23 antibody according to any one of the above, (i) in the heavy chain variable region, the HCDR1 comprises the amino acid sequence of SEQ ID NO: 27, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(ii) in the heavy chain variable region, the HCDR1 comprises the amino acid sequence of SEQ ID NO: 28, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
(iii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37, and the LCDR3 comprises the amino acid sequence of SEQ ID NO: 16.

In some embodiments of the anti-IL23 antibody according to any one of the above, the anti-IL23 antibody is a murine antibody, a chimeric antibody, or a humanized antibody. In some embodiments, the antibody is a humanized antibody.

In some embodiments of the anti-IL23 antibody according to any one of the above, the anti-IL23 antibody comprises a heavy chain variable region and a light chain variable region, wherein
(i) the heavy chain variable region comprises any one of SEQ ID NO: 22, 21, 20, 19, 18, or 17, or an amino acid sequence having at least 90% (e.g., at least 90%, 95%, 96%, 97%, 98%, or 99%) sequence identity thereto, and the light chain variable region comprises any one of SEQ ID NO: 26, 25, 24, or 23, or an amino acid sequence having at least 90% (e.g., at least 90%, 95%, 96%, 97%, 98%, or 99%) sequence identity thereto; or
(ii) the heavy chain variable region comprises any one of SEQ ID NO: 29, 30, 31, or 32 or an amino acid sequence having at least 90% (e.g., at least 90%, 95%, 96%, 97%, 98%, or 99%) sequence identity thereto, and the light chain variable region comprises any one of SEQ ID NO: 36, 35, 34, or 33, or an amino acid sequence having at least 90% (e.g., at least 90%, 95%, 96%, 97%, 98%, or 99%) sequence identity thereto; or
(iii) the heavy chain variable region comprises SEQ ID NO: 1 or an amino acid sequence having at least 90% (e.g., at least 90%, 95%, 96%, 97%, 98%, or 99%) sequence identity thereto, and the light chain variable region comprises SEQ ID NO: 2 or an amino acid sequence having at least 90% (e.g., at least 90%, 95%, 96%, 97%, 98%, or 99%) sequence identity thereto; or
(iv) the heavy chain variable region comprises SEQ ID NO: 3 or an amino acid sequence having at least 90% (e.g., at least 90%, 95%, 96%, 97%, 98%, or 99%) sequence identity thereto, and the light chain variable region comprises SEQ ID NO: 4 or an amino acid sequence having at least 90% (e.g., at least 90%, 95%, 96%, 97%, 98%, or 99%) sequence identity thereto.

In some embodiments of the anti-IL23 antibody according to any one of the above, in the anti-IL23 antibody, the heavy chain variable region comprises, in a framework region thereof, one or more amino acid mutations selected from the group consisting of positions 1, 30, 37, 44, 49, 73, 89, and 93 (numbering according to Kabat numbering scheme), and/or the light chain variable region comprises, in a framework region thereof, one or more amino acid mutations selected from the group consisting of positions 4, 17, 36, 58, 60, and 68 (numbering according to Kabat numbering scheme). In some embodiments, in the heavy chain variable region of the anti-IL23 antibody, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; in the antibody, the heavy chain variable region comprises, in a framework region thereof, one or more amino acid mutations selected from the group consisting of 1E, 30T, 37V, 44G, 49G, 73N, 89R, and 93V (numbering according to Kabat numbering scheme), and/or the light chain variable region comprises, in a framework region thereof, one or more amino acid mutations selected from the group consisting of 4L, 17Q, 36F, 58I, 60A, and 68R (numbering according to Kabat numbering scheme).

In some embodiments of the anti-IL23 antibody according to any one of the above, in the anti-IL23 antibody, the heavy chain variable region comprises, in a framework region thereof, one or more amino acid mutations selected from the group consisting of positions 1, 2, 28, 44, 71, and 73 (numbering according to Kabat numbering scheme), and/or the light chain variable region comprises, in a framework region thereof, one or more amino acid mutations selected from the group consisting of positions 4, 36, 39, 71, and 100 (numbering according to Kabat numbering scheme). In some embodiments, in the heavy chain variable region of the anti-IL23 antibody, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16; in the antibody, the heavy chain variable region comprises, in a framework region thereof, one or more amino acid mutations selected from the group consisting of 1E, 2F, 28S, 44S, 71V, and 73Q (numbering according to Kabat numbering scheme), and/or the light chain variable region comprises, in a framework region thereof, one or more amino acid mutations selected from the group consisting of 4I, 36F, 39R, 71L, and 100S (numbering according to Kabat numbering scheme).

In some embodiments, the anti-IL23 antibody according to any one of the above comprises a heavy chain variable region and a light chain variable region, wherein
(i) the heavy chain variable region comprises the amino acid sequence of SEQ ID NO: 22, 21, 20, 19, 18, or 17, and the light chain variable region comprises the amino acid sequence of SEQ ID NO: 26, 25, 24, or 23; or
(ii) the heavy chain variable region comprises the amino acid sequence of SEQ ID NO: 29, 30, 31, or 32, and the light chain variable region comprises the amino acid sequence of SEQ ID NO: 36, 35, 34, or 33; or
(iii) the heavy chain variable region comprises the amino acid sequence of SEQ ID NO: 1, and the light chain variable region comprises the amino acid sequence of SEQ ID NO: 2; or
(iv) the heavy chain variable region comprises the amino acid sequence of SEQ ID NO: 3, and the light chain variable region comprises the amino acid sequence of SEQ ID NO: 4.

In some embodiments, the anti-IL23 antibody according to any one of the above comprises a heavy chain variable region and a light chain variable region, wherein
(i) the heavy chain variable region comprises the amino acid sequence of SEQ ID NO: 22, and the light chain variable region comprises the amino acid sequence of SEQ ID NO: 26; or
(ii) the heavy chain variable region comprises the amino acid sequence of SEQ ID NO: 21, and the light chain variable region comprises the amino acid sequence of SEQ ID NO: 25; or
(iii) the heavy chain variable region comprises the amino acid sequence of SEQ ID NO: 29, and the light chain variable region comprises the amino acid sequence of SEQ ID NO: 36.

In some embodiments of the anti-IL23 antibody according to any one of the above, the anti-IL23 antibody further comprises antibody heavy and light chain constant regions. In some embodiments, the heavy chain constant region is a human IgG heavy chain constant region; in some embodiments, the heavy chain constant region is selected from the group consisting of human IgG 1, IgG2, IgG3, and IgG4 constant regions; in some embodiments, the light chain constant region is selected from the group consisting of human antibody κ and λ chain constant regions; in some embodiments, the heavy chain constant region is a human IgG1 heavy chain constant region, and the light chain constant region is a human κ light chain constant region. In some embodiments, the Fc region of the heavy chain constant region has one or more amino acid substitutions capable of reducing the binding of the Fc region to an Fc receptor. In some embodiments, the Fc region has L234A and L235A mutations, and/or S228P mutation, and/or YTE mutations (M252Y, S254T, and T256E), and the numbering of the mutations is according to the EU index. In some embodiments, the heavy chain constant region comprises the amino acid sequence of SEQ ID NO: 38, and the light chain constant region comprises the amino acid sequence of SEQ ID NO: 39.

In some embodiments, the anti-IL23 antibody according to any one of the above comprises a heavy chain and a light chain, wherein
(i) in the anti-IL23 antibody, the heavy chain comprises an amino acid sequence having at least 90% (e.g., at least 90%, 95%, 96%, 97%, 98%, or 99%) sequence identity to SEQ ID NO: 42, and the light chain comprises an amino acid sequence having at least 90% (e.g., at least 90%, 95%, 96%, 97%, 98%, or 99%) sequence identity to SEQ ID NO: 43; or
(ii) in the anti-IL23 antibody, the heavy chain comprises an amino acid sequence having at least 90% (e.g., at least 90%, 95%, 96%, 97%, 98%, or 99%) sequence identity to SEQ ID NO: 40, and the light chain comprises an amino acid sequence having at least 90% (e.g., at least 90%, 95%, 96%, 97%, 98%, or 99%) sequence identity to SEQ ID NO: 41; or
(iii) in the anti-IL23 antibody, the heavy chain comprises an amino acid sequence having at least 90% (e.g., at least 90%, 95%, 96%, 97%, 98%, or 99%) sequence identity to SEQ ID NO: 44, and the light chain comprises an amino acid sequence having at least 90% (e.g., at least 90%, 95%, 96%, 97%, 98%, or 99%) sequence identity to SEQ ID NO: 45.

In some embodiments, the anti-IL23 antibody according to any one of the above comprises a heavy chain and a light chain, wherein
(i) in the anti-IL23 antibody, the heavy chain comprises the amino acid sequence of SEQ ID NO: 42, and the light chain comprises the amino acid sequence of SEQ ID NO: 43; or
(ii) in the anti-IL23 antibody, the heavy chain comprises the amino acid sequence of SEQ ID NO: 40, and the light chain comprises the amino acid sequence of SEQ ID NO: 41; or
(iii) in the anti-IL23 antibody, the heavy chain comprises the amino acid sequence of SEQ ID NO: 44, and the light chain comprises the amino acid sequence of SEQ ID NO: 45.

In some embodiments, the present disclosure also provides an isolated anti-IL23 antibody or anti-IL23 antibody fusion protein that competes for binding to human IL23 with the anti-IL23 antibody or the anti-IL23 antibody fusion protein according to any one of the above.

In some embodiments, the anti-IL23 antibody or the anti-IL23 antibody fusion protein according to any one of the above has one or more of the following properties:
A. specifically binding to human IL-23 p19 and cynomolgus monkey IL-23 p19, but not specifically binding to mouse IL-23 p19; preferably, binding to human IL-23 p19 with a KD value of less than 6.00E-11 M (e.g., less than 6.00E-11 M, less than 5.00E-11 M, less than 4.00E-11 M, less than 3.00E-11 M, less than 2.00E-11 M, less than 1.7E-11 M, less than 1.6E-11 M, less than 1.5E-11 M, or less) and/or binding to cynomolgus monkey IL-23 p19 with a KD value of less than 9.00E-11 M (e.g., less than 9.00E-11 M, less than 8.00E-11 M, less than 7.00E-11 M, less than 6.00E-11 M, or less), the KD values being measured by a surface plasmon resonance assay, e.g., by BIACORE^{®} surface plasmon resonance assay, wherein in some embodiments, the KD values are measured by the method in Test Example 6 of the present disclosure;
B. having the activity of blocking IL-23/IL-23R binding; preferably, blocking human IL-23/IL-23R binding with an IC₅₀ value of less than 0.6 nM (e.g., less than 0.6 nM, less than 0.5 nM, less than 0.4 nM, less than 0.3 nM, or less), the IC₅₀ value being measured by Elisa, wherein in some embodiments, the IC₅₀ value is measured by the method in Test Example 2 of the present disclosure;
C. having the activity of blocking BAFF/BAFF-R binding; preferably, blocking BAFF/BAFF-R binding with an IC₅₀ value of less than 7.00 nM (e.g., less than 7.00 nM, less than 6.00 nM, less than 5.00 nM, less than 4.00 nM, less than 3.00 nM, less than 2.00 nM, less than 1.00 nM, less than 0.9 nM, or less), the IC₅₀ value being measured by Elisa, wherein in some embodiments, the IC₅₀ value is measured by the method in Test Example 2 of the present disclosure;
D. having the activity of blocking BAFF/BCMA binding; preferably, blocking BAFF/BCMA binding with an IC₅₀ value of less than 4.50 nM (e.g., less than 4.50 nM, less than 3.00 nM, less than 2.00 nM, less than 1.00 nM, or less), the IC₅₀ value being measured by Elisa, wherein in some embodiments, the IC₅₀ value is measured by the method in Test Example 2 of the present disclosure;
E. having the activity of blocking BAFF/TACI binding; preferably, blocking BAFF/TACI binding with an IC₅₀ value of less than 6.00 nM (e.g., less than 6.00 nM, less than 5.00 nM, less than 4.00 nM, less than 3.00 nM, less than 2.00 nM, less than 1.00 nM, or less), the IC₅₀ value being measured by Elisa, wherein in some embodiments, the IC₅₀ value is measured by the method in Test Example 2 of the present disclosure;
F. having the activity of blocking APRIL/BCMA binding; preferably, blocking APRIL/BCMA binding with an IC₅₀ value of less than 1.00 nM (e.g., less than 1.00 nM, less than 0.5 nM, less than 0.4 nM, less than 0.1 nM, or less), the IC₅₀ value being measured by Elisa, wherein in some embodiments, the IC₅₀ value is measured by the method in Test Example 2 of the present disclosure;
G. having the activity of blocking APRIL/TACI binding; preferably, blocking APRIL/TACI binding with an IC₅₀ value of less than 3.00 nM (e.g., less than 3.00 nM, less than 2.00 nM, less than 1.50 nM, or less), the IC₅₀ value being measured by Elisa, wherein in some embodiments, the IC₅₀ value is measured by the method in Test Example 2 of the present disclosure;
H. having the activity of inhibiting IL-17 secretion; preferably, inhibiting IL-17 secretion with an IC₅₀ value of less than 0.03 nM (e.g., less than 0.03 nM, less than 0.02 nM, less than 0.01 nM, or less), wherein in some embodiments, the IC₅₀ value is measured by the method in Test Example 4 of the present disclosure;
I. having the activity of inhibiting proliferation of BaF3-IL-23R cells; preferably, inhibiting proliferation of BaF3-IL-23R cells with an IC₅₀ value of less than 0.5 nM (e.g., less than 0.5 nM, less than 0.4 nM, less than 0.3 nM, less than 0.2 nM, less than 0.1 nM, or less), the IC₅₀ value being measured by PerkinElmer, wherein in some embodiments, the IC₅₀ value is measured by the method in Test Example 3 of the present disclosure;
J. having the activity of inhibiting secretion of cytokines TNFα, IL-22, and IgA, wherein in some embodiments, the TNFα, IL-22, and IgA are detected by the method in Test Example 9 of the present disclosure; or
K. having the activity of inhibiting proliferation of B cells, wherein in some embodiments, the TNFα, IL-22, and IgA are detected by the method in Test Example 5 of the present disclosure.

The present disclosure also provides a pharmaceutical composition comprising the anti-IL23 antibody fusion protein or the anti-IL23 antibody according to any one of the above, and one or more pharmaceutically acceptable carriers, diluents, or excipients.

In some embodiments, the present disclosure provides a nucleic acid molecule encoding the anti-IL23 antibody fusion protein or the anti-IL23 antibody according to any one of the above.

In some embodiments, the present disclosure provides an expression vector comprising the nucleic acid molecule described above.

In some embodiments, the present disclosure provides a host cell comprising the nucleic acid molecule described above.

In some embodiments, the present disclosure provides a host cell comprising the expression vector described above.

In some embodiments, the present disclosure provides a method for treating or ameliorating a B cell disorder or an autoimmune disease, which comprises the step of administering to a subject in need thereof a therapeutically effective amount of the anti-IL23 antibody fusion protein or the anti-IL23 antibody, or the pharmaceutical composition according to any one of the above.

In some embodiments, the present disclosure provides use of the anti-IL23 antibody fusion protein or the anti-IL23 antibody, the nucleic acid molecule, or the pharmaceutical composition according to any one of the above in the preparation of a medicament for treating or preventing a disease.

In another aspect, the present disclosure also provides the anti-IL23 antibody fusion protein or the anti-IL23 antibody, the nucleic acid molecule, or the composition according to any one of the above for use as a medicament. In some embodiments, the medicament is used for treating a B cell disorder or an autoimmune disease.

In some embodiments, the disease according to any one of the above is a disease or condition associated with IL23 expression. In some embodiments, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of tumors, chronic leukocytic leukemia, multiple myeloma, non-Hodgkin's lymphoma, post-transplant lymphoproliferative disorder, and light chain gammopathy. In some embodiments, the disease is systemic lupus erythematosus.

In some embodiments, the treatment according to any one of the above further comprises administering to the subject an additional therapeutic drug.

### BRIEF DESCRIPTION OF THE DRAWINGS

FIG. 1: schematic structural diagram of Hu29-19T and Hu29-24T;
FIG. 2: experimental results of scores for the severity of erythema in imiquimod-induced psoriasis animals;
FIG. 3: experimental results of scores for the severity of skin scaling in imiquimod-induced psoriasis animals;
FIG. 4: the comprehensive scoring results for erythema and skin scaling in imiquimod-induced psoriasis animals;
FIG. 5: experimental results of the thickness of the right ears of human IL-23-induced psoriasis animals;
FIG. 6: experimental results of the area under curve of human IL-23-induced psoriasis animals (in the figure, *** P < 0.001 compared with negative control);
FIG. 7: experimental results of the weight of right ears of human IL-23-induced psoriasis animals (in the figure, ** P < 0.01 and *** P < 0.001 compared with negative control);
FIG. 8: experimental results of the inhibition of IgA secretion by anti-IL23 antibody fusion proteins;
FIG. 9: experimental results of the inhibition of TNFα secretion by anti-IL23 antibody fusion proteins;
FIG. 10: experimental results of the inhibition of IL-22 secretion by anti-IL23 antibody fusion proteins;
FIG. 11: renal pathological score results in a pharmacodynamic experiment of animals with systemic lupus erythematosus;
FIG. 12: skin damage score results in a pharmacodynamic experiment of animals with systemic lupus erythematosus;
FIG. 13: urine protein detection results in a pharmacodynamic experiment of animals with systemic lupus erythematosus.

### DETAILED DESCRIPTION

### Terminology

To facilitate the understanding of the present disclosure, some technical and scientific terms are described below. Unless otherwise specifically defined herein, all technical and scientific terms used herein have the same meaning as commonly understood by those of ordinary skill in the art.

As used in the description and in the claims, the singular forms of "a", "an", and "the" include plural referents unless otherwise clearly indicated in the context.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include: IL-2, IFN-γ, IL-6, TNFα, IL-17, and IL-5.

TACI described herein is a membrane-bound receptor, and the wild-type human TACI comprises an extracellular region comprising two cysteine-rich pseudo-repeats, a transmembrane region, and a cytoplasmic region that interacts with CAML (calcium-modulating cyclophilin ligand). The extracellular region (positions 1 to 165) of the wild-type human TACI is set forth in SEQ ID NO: 51 in the present disclosure. "TACI extracellular domain" and "TACI extracellular region" herein are used interchangeably. IL-23 (also known as IL23) is mainly produced by activated dendritic cells, macrophages, monocytes, etc. It is a member of the IL-12 heterodimer cytokine family and consists of two subunits, p19 (also known as IL23 p19 subunit) and p40 (also known as IL23 p40 subunit), wherein the p40 subunit is a subunit also contained in IL-12 (J Immunol. 2018 Sep 15; 201(6): 1605-1613).

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used herein are as described in J. Biol. Chem. 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W. The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Natural antibody" refers to a naturally-occurring immunoglobulin molecule. For example, a native IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by heavy chain constant regions. Generally, a native IgG heavy chain constant region comprises three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "full-length antibody", "intact antibody", and "whole antibody" are used herein interchangeably and refer to an antibody having a substantially similar structure to a native antibody structure or having heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), Sequences of Proteins of Immunological Interest, 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are, e.g., as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, F(ab')₂, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" antibody refers to an antibody in which a portion of the heavy and/or light chain in the antibody is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable and constant regions are human sequences. The term encompasses antibodies that are derived from human genes but have, for example, sequences that have been altered to, e.g., reduce possible immunogenicity, increase affinity, eliminate cysteines or glycosylation sites that may cause undesired folding. The term encompasses antibodies recombinantly produced in non-human cells (that may confer glycosylation not characteristic of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1: 1 interaction between members of a binding pair (e.g., an antibody and an antigen). The affinity of a molecule X for its ligand Y can be generally denoted by the dissociation constant (KD). Affinity can be determined by conventional methods known in the art, including those described herein.

As used herein, the term "k_{assoc}" or "kₐ" refers to the association rate of a particular antibody-antigen interaction, while the term "k_{dis}" or "k_{d}" refers to the dissociation rate of a particular antibody-antigen interaction. The term "KD" refers to the dissociation constant, which is obtained from the ratio of k_{d} to kₐ (i.e., k_{d}/kₐ) and denoted by a molar concentration (M). The KD value of an antibody can be determined using methods well known in the art. For example, surface plasmon resonance is determined using a biosensing system such as a system (e.g., Biacore), or affinity in a solution is determined by solution equilibrium titration (SET).

The term "effector function" refers to biological activities that can be attributed to the Fc region of an antibody (either the native sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or portion of a molecule that is capable of being bound by a selective binding agent, such as an antigen-binding protein (including, for example, an antibody), and that is also capable of being used in an animal to produce an antibody that is capable of binding to the antigen. An antigen may have one or more epitopes capable of interacting with different antigen-binding proteins (e.g., antibodies). The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to the same epitope) can be performed using routine methods in the art, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody is capable of binding to a certain antigen or an epitope in the antigen with higher affinity than to other antigens or epitopes. Generally, an antibody binds to an antigen or an epitope in the antigen with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by BIACORE^{®} surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The terms "anti-IL23 antibody" and "antibody that binds to II,23" refer to an antibody that is capable of binding to IL23 with sufficient affinity. In one embodiment, the degree of the binding of an antibody to an unrelated and non-IL23 protein is less than about 10% of the binding of the antibody to IL23 and IL12, as measured by BIACORE^{®} surface plasmon resonance assay. In certain embodiments, an antibody that binds to IL23 protein has a dissociation constant (KD) of < about 1 µM, < about 100 nM, < about 10 nM, < about 1 nM, < about 0.1 nM, < about 0.01 nM, or < about 0.001 nM. In certain embodiments, the anti-IL23 antibody binds to an epitope of the human or cynomolgus monkey IL23 p19 subunit.

The term "linker" refers to a linker unit that links two polypeptide fragments and generally has some flexibility, such that the use of the linker does not result in the loss of the original function of the protein domain. Herein, the linkers present in the same structure may be identical or different. The linker may be a peptide linker comprising one or more amino acids, typically about 1-30, 2-24, or 3-15 amino acids. The linkers used herein may be identical or different.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcy receptor (FcyR) expressed on the effector cells. For example, NK cells express FcyRIIIa, while monocytes express FcyRI, FcyRII, and FcyRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

In the present disclosure, for example, "a position relative to a XX sequence" refers to a position of a test sequence corresponding to that of a XX sequence, i.e., a relative position of the two sequences, when the test sequence is optimally aligned with the XX sequence to obtain the highest percent identity. For example, amino acid residue positions, numbered in natural order, in the extracellular region sequences SEQ ID NO: 51 and SEQ ID NO: 60 of TACI relative to SEQ ID NO: 58 are shown in Table 2.

**Table 2. Positions in SEQ ID NO: 58 and corresponding positions in SEQ ID NO: 51/SEQ ID NO: 60**

| Positions in SEQ ID NO: 58 (in natural order) | Corresponding position | |
|---|---|---|
| | Corresponding positions in SEQ ID NO: 51 (in natural order) | Corresponding positions in SEQ ID NO: 60 (in natural order) |
| Positions 48-88 | Positions 68-108 | Positions 1-41 |
| Positions 48-87 | Positions 68-107 | Positions 1-40 |
| Positions 48-86 | Positions 68-106 | Positions 1-39 |
| Positions 48-85 | Positions 68-105 | Positions 1-38 |
| Position 49 | Position 69 | Position 2 |
| Position 52 | Position 72 | Position 5 |
| Position 53 | Position 73 | Position 6 |
| Position 57 | Position 77 | Position 10 |
| Position 65 | Position 85 | Position 18 |
| Position 82 | Position 102 | Position 35 |
| Position 83 | Position 103 | Position 36 |

| | | |
|---|---|---|
| Note: for example, the residue position 2 (in natural order) in SEQ ID NO: 60 and the residue position 49 in SEQ ID NO: 58 (in natural order) are corresponding positions. The term "fusion" or "linkage" means that components (e.g., the TACI polypeptide and the heavy chain of antibody) are linked directly or by a covalent bond via one or more linkers. When the linker is a peptide linker, the covalent bond is a peptide bond. | | |

The term "anti-IL23 antibody fusion protein" refers to a protein in which an anti-IL23 antibody is fused to an active protein. For example, a TACI polypeptide is fused to the terminus of the heavy chain of an anti-IL23 antibody to form a protein.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be completely identical to parent cells in terms of nucleic acid content and may contain mutations. Mutant progenies that have the same function or biological activity as the cells screened or selected from the group consisting of the initially transformed cells are included herein. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris*, *Pichia finlandica*, *Pichia trehalophila*, *Pichia koclamae*, *Pichia membranaefaciens, Pichia minuta (Ogataea minuta, Pichia lindneri), Pichiaopuntiae, Pichia thermotolerans, Pichia salictaria*, *Pichia guercuum, Pichia pijperi*, *Pichia stiptis, Pichia methanolica, Pichia, Saccharomycescerevisiae*, *Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa. Pichia,* any *Saccharomyces, Hansenula polymorpha,* any *Kluyveromyces, Candida albicans,* any *Aspergillus*, *Trichoderma reesei, Chrysosporium lucknowense*, any *Fusarium, Yarrowia lipolytica,* and *Neurospora crassa.*

As used herein, the expressions "cell", "cell line", and "cell culture" are used interchangeably, and all such designations include their progenies. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the same function or biological activity as the original transformed cell from which they were selected are included.

"Optional" or "optionally" means that the event or circumstance subsequently described may, but does not necessarily, occur, and that the description includes instances where the event or circumstance occurs or does not occur.

The term "pharmaceutical composition" refers to a mixture comprising one or more of the anti-IL23 antibody fusion proteins described herein and other chemical components, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subj ect" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some embodiments, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

### Anti-IL23 Antibody of the Present Disclosure

In one aspect, the present disclosure designs a novel anti-IL23 antibody and a fusion protein thereof. The antibody has one or more of the following properties:
A. specifically binding to human IL-23 p19 and cynomolgus monkey IL-23 p19, but not specifically binding to mouse IL-23 p19; preferably, binding to human IL-23 p19 with a KD value of less than 6.00E-11 M (e.g., less than 6.00E-11 M, less than 5.00E-11 M, less than 4.00E-11 M, less than 3.00E-11 M, less than 2.00E-11 M, less than 1.7E-11 M, less than 1.6E-11 M, less than 1.5E-11 M, or less) and/or binding to cynomolgus monkey IL-23 p19 with a KD value of less than 9.00E-11 M (e.g., less than 9.00E-11 M, less than 8.00E-11 M, less than 7.00E-11 M, less than 6.00E-11 M, or less), the KD values being measured by a surface plasmon resonance assay, e.g., by BIACORE^{®} surface plasmon resonance assay, wherein in some embodiments, the detection
B. having the activity of blocking IL-23/IL-23R binding; preferably, blocking human IL-23/IL-23R binding with an IC₅₀ value of less than 0.6 nM (e.g., less than 0.6 nM, less than 0.5 nM, less than 0.4 nM, less than 0.3 nM, or less), the IC₅₀ value being measured by Elisa;
C. having the activity of inhibiting IL-17 secretion; preferably, inhibiting IL-17 secretion with an IC₅₀ value of less than 0.03 nM (e.g., less than 0.03 nM, less than 0.02 nM, less than 0.01 nM, or less), the IC₅₀ value being measured by Elisa;
D. having the activity of inhibiting proliferation of BaF3-IL-23R cells; preferably, inhibiting proliferation of BaF3-IL-23R cells with an IC₅₀ value of less than 0.5 nM (e.g., less than 0.5 nM, less than 0.4 nM, less than 0.3 nM, less than 0.2 nM, less than 0.1 nM, or less), the IC₅₀ value being measured by PerkinElmer;
E. having the activity of inhibiting TNFα and/or IL-22 secretion; or
F. having strong *in vivo* efficacy in an animal model of imiquimod-induced psoriasis, an animal model of human IL-23-induced psoriasis, and/or an animal model of systemic lupus erythematosus.

### Exemplary Anti-IL23 Antibodies

In one aspect, the present disclosure provides an anti-IL23 antibody, wherein the anti-IL23 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein in some embodiments, in the anti-IL23 antibody according to any one of the above, the HCDR1, the HCDR2, and the HCDR3 of the heavy chain variable region and the LCDR1, the LCDR2, and the LCDR3 of the light chain variable region are defined according to the Kabat numbering scheme, wherein
(i) in the heavy chain variable region, the HCDR1 is set forth in SEQ ID NO: 27, 28, or 5, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(ii) in the heavy chain variable region, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37 or 15, and the LCDR3 is set forth in SEQ ID NO: 16.

In some embodiments, the anti-IL23 antibody according to any one of the above comprises a heavy chain variable region and a light chain variable region, wherein
(i) in the heavy chain variable region, the HCDR1 is set forth in SEQ ID NO: 27, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(ii) in the heavy chain variable region, the HCDR1 is set forth in SEQ ID NO: 28, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(iii) in the heavy chain variable region, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37, and the LCDR3 is set forth in SEQ ID NO: 16.

In some embodiments, the anti-IL23 antibody according to any one of the above is murine, chimeric, or humanized. In some embodiments, the anti-IL23 antibody is humanized. In some embodiments, in the anti-IL23 antibody, the heavy chain variable region has a FR1, a FR2, and a FR3 derived from IGHV2-26*01 and a FR4 derived from IGHJ6*01, which are unsubstituted or have one or more amino acid substitutions selected from the group consisting of 1E, 30T, 37V, 44G, 49G, 73N, 89R, and 93V; and/or the light chain variable region has a FR1, a FR2, and a FR3 derived from IGKV4-1*01 and a FR4 derived from IGKJ4*01, which are unsubstituted or have one or more amino acid substitutions selected from the group consisting of 4L, 17Q, 36F, 58I, 60A, and 68R. In some embodiments, in the heavy chain variable region of the anti-IL23 antibody, the HCDR1 is set forth in SEQ ID NO: 27, 28, or 5, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10. The variable regions and CDRs described above are defined according to the Kabat numbering scheme.

In some embodiments, the anti-IL23 antibody according to any one of the above is a humanized antibody. In some embodiments, in the anti-IL23 antibody, the heavy chain variable region has a FR1, a FR2, and a FR3 derived from IGHV1-3*01 and a FR4 derived from IGHJ1*01, which are unsubstituted or have one or more amino acid substitutions selected from the group consisting of 1E, 2F, 28S, 44S, 71V, and 73Q; and/or the light chain variable region has a FR1, a FR2, and a FR3 derived from IGKV2-40*01 and a FR4 derived from IGKJ2*01, which are unsubstituted or have one or more amino acid substitutions selected from the group consisting of 4I, 36F, 39R, 71L, and 100S. In some embodiments, in the heavy chain variable region of the anti-IL23 antibody, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37 or 15, and the LCDR3 is set forth in SEQ ID NO: 16. The variable regions and CDRs described above are defined according to the Kabat numbering scheme.

In some embodiments, the anti-IL23 antibody according to any one of the above comprises a heavy chain variable region and a light chain variable region, wherein
(i) the heavy chain variable region is set forth in SEQ ID NO: 22, 21, 20, 19, 18, or 17, and the light chain variable region is set forth in SEQ ID NO: 26, 25, 24, or 23; or
(ii) the heavy chain variable region is set forth in SEQ ID NO: 29, 30, 31, or 32, and the light chain variable region is set forth in SEQ ID NO: 36, 35, 34, or 33; or
(iii) the heavy chain variable region is set forth in SEQ ID NO: 1, and the light chain variable region is set forth in SEQ ID NO: 2; or
(iv) the heavy chain variable region is set forth in SEQ ID NO: 3, and the light chain variable region is set forth in SEQ ID NO: 4.

In some embodiments, the anti-IL23 antibody according to any one of the above comprises a heavy chain variable region and a light chain variable region, wherein
(i) the heavy chain variable region is set forth in SEQ ID NO: 22, and the light chain variable region is set forth in SEQ ID NO: 26;
(ii) the heavy chain variable region is set forth in SEQ ID NO: 21, and the light chain variable region is set forth in SEQ ID NO: 25; or
(iii) the heavy chain variable region is set forth in SEQ ID NO: 29, and the light chain variable region is set forth in SEQ ID NO: 36.

In some embodiments, the anti-IL23 antibody according to any one of the above further comprises an antibody heavy and light chain constant regions. In some embodiments, the heavy chain constant region is a human IgG heavy chain constant region; in some embodiments, the heavy chain constant region is selected from the group consisting of human IgG1, IgG2, IgG3, and IgG4 constant regions; in some embodiments, the light chain constant region is selected from the group consisting of human antibody κ and λ chain constant regions; in some embodiments, the heavy chain constant region is a human IgG1 heavy chain constant region, and the light chain constant region is a human κ light chain constant region. In some embodiments, the Fc region of the heavy chain constant region has one or more amino acid substitutions capable of reducing the binding of the Fc region to an Fc receptor. In some embodiments, the Fc region has L234A and L235A mutations, and/or S228P mutation, and/or YTE mutations (M252Y, S254T, and T256E), and the numbering of the mutations is according to the EU index. In some embodiments, the Fc region has L234A and L235A mutations. In some embodiments, the heavy chain constant region comprises the amino acid sequence of SEQ ID NO: 38, and the light chain constant region comprises the amino acid sequence of SEQ ID NO: 39.

In some embodiments, the anti-IL23 antibody according to any one of the above comprises a heavy chain and a light chain, wherein
(i) in the anti-IL23 antibody, the heavy chain comprises an amino acid sequence having at least 90% (e.g., at least 90%, 95%, 96%, 97%, 98%, or 99%) sequence identity to SEQ ID NO: 42, and the light chain comprises an amino acid sequence having at least 90% (e.g., at least 90%, 95%, 96%, 97%, 98%, or 99%) sequence identity to SEQ ID NO: 43; or
(ii) in the anti-IL23 antibody, the heavy chain comprises an amino acid sequence having at least 90% (e.g., at least 90%, 95%, 96%, 97%, 98%, or 99%) sequence identity to SEQ ID NO: 40, and the light chain comprises an amino acid sequence having at least 90% (e.g., at least 90%, 95%, 96%, 97%, 98%, or 99%) sequence identity to SEQ ID NO: 41; or
(iii) in the anti-IL23 antibody, the heavy chain comprises an amino acid sequence having at least 90% (e.g., at least 90%, 95%, 96%, 97%, 98%, or 99%) sequence identity to SEQ ID NO: 44, and the light chain comprises an amino acid sequence having at least 90% (e.g., at least 90%, 95%, 96%, 97%, 98%, or 99%) sequence identity to SEQ ID NO: 45.

In some embodiments, the anti-IL23 antibody according to any one of the above comprises a heavy chain and a light chain, wherein
(i) in the anti-IL23 antibody, the heavy chain is set forth in SEQ ID NO: 42, and the light chain is set forth in SEQ ID NO: 43; or
(ii) in the anti-IL23 antibody, the heavy chain is set forth in SEQ ID NO: 40, and the light chain is set forth in SEQ ID NO: 41; or
(iii) in the anti-IL23 antibody, the heavy chain is set forth in SEQ ID NO: 44, and the light chain is set forth in SEQ ID NO: 45.

### Anti-IL23 Antibody Fusion Protein of the Present Disclosure

The present disclosure constructs an anti-IL23 antibody fusion protein comprising an anti-IL23 antibody and a TACI polypeptide, wherein the anti-IL23 antibody specifically binds to human IL23, preferably specifically binds to human IL23 p19 subunit. In some embodiments, the anti-IL23 antibody or the anti-IL23 antibody fusion protein according to any one of the above has one or more of the following properties:
A. specifically binding to human IL-23 p19 and cynomolgus monkey IL-23 p19, but not specifically binding to mouse IL-23 p19; preferably, binding to human IL-23 p19 with a KD value of less than 6.00E-11 M (e.g., less than 6.00E-11 M, less than 5.00E-11 M, less than 4.00E-11 M, less than 3.00E-11 M, less than 2.00E-11 M, less than 1.7E-11 M, less than 1.6E-11 M, less than 1.5E-11 M, or less) and/or binding to cynomolgus monkey IL-23 p19 with a KD value of less than 9.00E-11 M (e.g., less than 9.00E-11 M, less than 8.00E-11 M, less than 7.00E-11 M, less than 6.00E-11 M, or less), the KD values being measured by a surface plasmon resonance assay, e.g., by BIACORE^{®} surface plasmon resonance assay;
B. having the activity of blocking IL-23/IL-23R binding; preferably, blocking human IL-23/IL-23R binding with an IC₅₀ value of less than 0.6 nM (e.g., less than 0.6 nM, less than 0.5 nM, less than 0.4 nM, less than 0.3 nM, or less), the IC₅₀ value being measured by Elisa;
C. having the activity of blocking BAFF/BAFF-R binding; preferably, blocking BAFF/BAFF-R binding with an IC₅₀ value of less than 7.00 nM (e.g., less than 7.00 nM, less than 6.00 nM, less than 5.00 nM, less than 4.00 nM, less than 3.00 nM, less than 2.00 nM, less than 1.00 nM, less than 0.9 nM, or less), the IC₅₀ value being measured by Elisa;
D. having the activity of blocking BAFF/BCMA binding; preferably, blocking BAFF/BCMA binding with an IC₅₀ value of less than 4.50 nM (e.g., less than 4.50 nM, less than 3.00 nM, less than 2.00 nM, less than 1.00 nM, or less), the IC₅₀ value being measured by Elisa;
E. having the activity of blocking BAFF/TACI binding; preferably, blocking BAFF/TACI binding with an IC₅₀ value of less than 6.00 nM (e.g., less than 6.00 nM, less than 5.00 nM, less than 4.00 nM, less than 3.00 nM, less than 2.00 nM, less than 1.00 nM, or less), the IC₅₀ value being measured by Elisa;
F. having the activity of blocking APRIL/BCMA binding; preferably, blocking APRIL/BCMA binding with an IC₅₀ value of less than 1.00 nM (e.g., less than 1.00 nM, less than 0.5 nM, less than 0.4 nM, less than 0.1 nM, or less), the IC₅₀ value being measured by Elisa;
G. having the activity of blocking APRIL/TACI binding; preferably, blocking APRIL/TACI binding with an IC₅₀ value of less than 3.00 nM (e.g., less than 3.00 nM, less than 2.00 nM, less than 1.50 nM, or less), the IC₅₀ value being measured by Elisa;
H. having the activity of inhibiting IL-17 secretion; preferably, inhibiting IL-17 secretion with an IC₅₀ value of less than 0.03 nM (e.g., less than 0.03 nM, less than 0.02 nM, less than 0.01 nM, or less), the IC₅₀ value being measured by Elisa;
I. having the activity of inhibiting proliferation of BaF3-IL-23R cells; preferably, inhibiting proliferation of BaF3-IL-23R cells with an IC₅₀ value of less than 0.5 nM (e.g., less than 0.5 nM, less than 0.4 nM, less than 0.3 nM, less than 0.2 nM, less than 0.1 nM, or less), the IC₅₀ value being measured by PerkinElmer;
J. having the activity of inhibiting secretion of cytokines TNFα, IL-22, and IgA; or
K. having the activity of inhibiting proliferation of B cells.

### Exemplary Anti-IL23 Antibody Fusion Proteins

In some embodiments of the anti-IL23 antibody fusion protein, the anti-IL23 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein the HCDR1, the HCDR2, and the HCDR3 of the heavy chain variable region and the LCDR1, the LCDR2, and the LCDR3 of the light chain variable region are defined according to the Kabat numbering scheme, wherein
(i) in the heavy chain variable region, the HCDR1 is set forth in SEQ ID NO: 27, 28, or 5, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(ii) in the heavy chain variable region, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37 or 15, and the LCDR3 is set forth in SEQ ID NO: 16.

In some embodiments,
(i) in the heavy chain variable region, the HCDR1 is set forth in SEQ ID NO: 27, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(ii) in the heavy chain variable region, the HCDR1 is set forth in SEQ ID NO: 28, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10; or
(iii) in the heavy chain variable region, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37, and the LCDR3 is set forth in SEQ ID NO: 16.

In some embodiments of the anti-IL23 antibody fusion protein according to any one of the above, the anti-IL23 antibody is a murine antibody, a chimeric antibody, or a humanized antibody. In some embodiments, the anti-IL23 antibody is a humanized antibody. In some embodiments, in the anti-IL23 antibody, the heavy chain variable region has a FR1, a FR2, and a FR3 derived from IGHV2-26*01 and a FR4 derived from IGHJ6*01, which are unsubstituted or have one or more amino acid substitutions selected from the group consisting of 1E, 30T, 37V, 44G, 49G, 73N, 89R, and 93V; and/or the light chain variable region has a FR1, a FR2, and a FR3 derived from IGKV4-1*01 and a FR4 derived from IGKJ4*01, which are unsubstituted or have one or more amino acid substitutions selected from the group consisting of 4L, 17Q, 36F, 58I, 60A, and 68R. In some embodiments, in the heavy chain variable region of the anti-IL23 antibody, the HCDR1 is set forth in SEQ ID NO: 27, 28, or 5, the HCDR2 is set forth in SEQ ID NO: 6, and the HCDR3 is set forth in SEQ ID NO: 7; and in the light chain variable region, the LCDR1 is set forth in SEQ ID NO: 8, the LCDR2 is set forth in SEQ ID NO: 9, and the LCDR3 is set forth in SEQ ID NO: 10. The variable regions and CDRs described above are defined according to the Kabat numbering scheme.

In some embodiments of the anti-IL23 antibody fusion protein according to any one of the above, the anti-IL23 antibody is a humanized antibody; in the anti-IL23 antibody, the heavy chain variable region has a FR1, a FR2, and a FR3 derived from IGHV1-3*01 and a FR4 derived from IGHJ1*01, which are unsubstituted or have one or more amino acid substitutions selected from the group consisting of 1E, 2F, 28S, 44S, 71V, and 73Q; and/or the light chain variable region has a FR1, a FR2, and a FR3 derived from IGKV2-40*01 and a FR4 derived from IGKJ2*01, which are unsubstituted or have one or more amino acid substitutions selected from the group consisting of 4I, 36F, 39R, 71L, and 100S. In some embodiments, in the heavy chain variable region of the anti-IL23 antibody, the HCDR1 is set forth in SEQ ID NO: 11, the HCDR2 is set forth in SEQ ID NO: 12, and the HCDR3 is set forth in SEQ ID NO: 13; and in the light chain variable region, the LCDR1 is set forth in SEQ ID NO: 14, the LCDR2 is set forth in SEQ ID NO: 37 or 15, and the LCDR3 is set forth in SEQ ID NO: 16. The variable regions and CDRs described above are defined according to the Kabat numbering scheme.

In some embodiments of the anti-IL23 antibody fusion protein according to any one of the above, the anti-IL23 antibody comprises a heavy chain variable region and a light chain variable region, wherein
(i) the heavy chain variable region is set forth in SEQ ID NO: 22, 21, 20, 19, 18, or 17, and the light chain variable region is set forth in SEQ ID NO: 26, 25, 24, or 23; or
(ii) the heavy chain variable region is set forth in SEQ ID NO: 29, 30, 31, or 32, and the light chain variable region is set forth in SEQ ID NO: 36, 35, 34, or 33; or
(iii) the heavy chain variable region is set forth in SEQ ID NO: 1, and the light chain variable region is set forth in SEQ ID NO: 2; or
(iv) the heavy chain variable region is set forth in SEQ ID NO: 3, and the light chain variable region is set forth in SEQ ID NO: 4.

In some embodiments of the anti-IL23 antibody fusion protein according to any one of the above, the anti-IL23 antibody comprises a heavy chain variable region and a light chain variable region, wherein
(i) the heavy chain variable region is set forth in SEQ ID NO: 22, and the light chain variable region is set forth in SEQ ID NO: 26; or
(ii) the heavy chain variable region is set forth in SEQ ID NO: 21, and the light chain variable region is set forth in SEQ ID NO: 25; or
(iii) the heavy chain variable region is set forth in SEQ ID NO: 29, and the light chain variable region is set forth in SEQ ID NO: 36.

In some embodiments of the anti-IL23 antibody fusion protein according to any one of the above, the anti-IL23 antibody further comprises antibody heavy and light chain constant regions. In some embodiments, the heavy chain constant region is a human IgG heavy chain constant region; in some embodiments, the heavy chain constant region is selected from the group consisting of human IgG1, IgG2, IgG3, and IgG4 constant regions; in some embodiments, the light chain constant region is selected from the group consisting of human antibody κ and λ chain constant regions; in some embodiments, the heavy chain constant region is a human IgG1 heavy chain constant region, and the light chain constant region is a human κ light chain constant region. In some embodiments, the Fc region of the heavy chain constant region has one or more amino acid substitutions capable of reducing the binding of the Fc region to an Fc receptor. In some embodiments, the Fc region has L234A and L235A mutations, and/or S228P mutation, and/or YTE mutations (M252Y, S254T, and T256E), and the numbering of the mutations is according to the EU index. In some embodiments, the heavy chain constant region comprises the amino acid sequence of SEQ ID NO: 38, and the light chain constant region comprises the amino acid sequence of SEQ ID NO: 39.

In some embodiments of the anti-IL23 antibody fusion protein according to any one of the above, the anti-IL23 antibody comprises a heavy chain and a light chain, wherein
(i) in the anti-IL23 antibody, the heavy chain comprises an amino acid sequence having at least 90% (e.g., at least 90%, 95%, 96%, 97%, 98%, or 99%) sequence identity to SEQ ID NO: 42, and the light chain comprises an amino acid sequence having at least 90% (e.g., at least 90%, 95%, 96%, 97%, 98%, or 99%) sequence identity to SEQ ID NO: 43; or
(ii) in the anti-IL23 antibody, the heavy chain comprises an amino acid sequence having at least 90% (e.g., at least 90%, 95%, 96%, 97%, 98%, or 99%) sequence identity to SEQ ID NO: 40, and the light chain comprises an amino acid sequence having at least 90% (e.g., at least 90%, 95%, 96%, 97%, 98%, or 99%) sequence identity to SEQ ID NO: 41; or
(iii) in the anti-IL23 antibody, the heavy chain comprises an amino acid sequence having at least 90% (e.g., at least 90%, 95%, 96%, 97%, 98%, or 99%) sequence identity to SEQ ID NO: 44, and the light chain comprises an amino acid sequence having at least 90% (e.g., at least 90%, 95%, 96%, 97%, 98%, or 99%) sequence identity to SEQ ID NO: 45.

In some embodiments of the anti-IL23 antibody fusion protein according to any one of the above, the anti-IL23 antibody comprises a heavy chain and a light chain, wherein
(i) in the anti-IL23 antibody, the heavy chain is set forth in SEQ ID NO: 42, and the light chain is set forth in SEQ ID NO: 43; or
(ii) in the anti-IL23 antibody, the heavy chain is set forth in SEQ ID NO: 40, and the light chain is set forth in SEQ ID NO: 41; or
(iii) in the anti-IL23 antibody, the heavy chain is set forth in SEQ ID NO: 44, and the light chain is set forth in SEQ ID NO: 45.

In some embodiments of the anti-IL23 antibody fusion protein according to any one of the above, the TACI polypeptide is less likely to break than a wild-type TACI polypeptide (the sequence being set forth in SEQ ID NO: 51).

In some embodiments of the anti-IL23 antibody fusion protein according to any one of the above, the TACI polypeptide is set forth in SEQ ID NO: 58 or a truncated fragment of SEQ ID NO: 58 or a variant thereof, wherein the truncated fragment comprises amino acid residues at positions 48-85 of SEQ ID NO: 58, and the variant is a variant having one or more amino acid replacements at positions selected from the group consisting of positions 49, 52, 53, 57, 65, 82, and 83 on the basis of SEQ ID NO: 58 or the truncated fragment thereof, wherein the position for the amino acid replacement is an amino acid residue position numbered in natural order relative to the sequence SEQ ID NO: 58.

In some embodiments of the anti-IL23 antibody fusion protein according to any one of the above, the truncated fragment of the TACI polypeptide comprises amino acid residues at positions 48-86 of SEQ ID NO: 58, amino acid residues at positions 48-87 of SEQ ID NO: 58, or amino acid residues at positions 48-88 of SEQ ID NO: 58.

In some embodiments of the anti-IL23 antibody fusion protein according to any one of the above, the TACI polypeptide has the sequence set forth in any one of SEQ ID NOs: 60-63.

In some embodiments of the anti-IL23 antibody fusion protein according to any one of the above, the sequence of the TACI polypeptide is a variant of SEQ ID NO: 58 or a truncated fragment (e.g., SEQ ID NO: 60, SEQ ID NO: 61, SEQ ID NO: 62, or SEQ ID NO: 63) of SEQ ID NO: 58, wherein the variant is a variant having any 1, 2, 3, 4, 5, 6, or 7 amino acid replacements at positions selected from the group consisting of positions 49, 52, 53, 57, 65, 82, and 83 on the basis of the sequence of SEQ ID NO: 58 or the truncated fragment thereof, wherein the position for the amino acid replacement is an amino acid residue position numbered in natural order relative to the sequence SEQ ID NO: 58.

In some embodiments of the anti-IL23 antibody fusion protein according to any one of the above, the TACI polypeptide is one having one or more amino acid replacements (e.g., 1, 2, 3, 4, 5, 6, or 7 amino acid replacements) selected from the group consisting of 49T or 49R, 52S, 53E or 53Q, 57E, 65T or 65A, 82A or 82R, and 83Y on the basis of the sequence of SEQ ID NO: 58, SEQ ID NO: 60, SEQ ID NO: 61, SEQ ID NO: 62, or SEQ ID NO: 63, wherein the position for the amino acid replacement is an amino acid residue position numbered in natural order relative to the sequence SEQ ID NO: 58.

In some embodiments of the anti-IL23 antibody fusion protein according to any one of the above, the TACI polypeptide has: any amino acid replacement selected from the group consisting of 49T, 52S, 53E, 53Q, 57E, and 82A on the basis of the sequence of SEQ ID NO: 58, SEQ ID NO: 60, SEQ ID NO: 61, SEQ ID NO: 62, or SEQ ID NO: 63; amino acid replacements 49R and 65T on the basis of the sequence of SEQ ID NO: 58, SEQ ID NO: 60, SEQ ID NO: 61, SEQ ID NO: 62, or SEQ ID NO: 63; amino acid replacements 45R and 65A on the basis of the sequence of SEQ ID NO: 58, SEQ ID NO: 60, SEQ ID NO: 61, SEQ ID NO: 62, or SEQ ID NO: 63; amino acid replacements 49R, 65T, and 82R on the basis of the sequence of SEQ ID NO: 58, SEQ ID NO: 60, SEQ ID NO: 61, SEQ ID NO: 62, or SEQ ID NO: 63; amino acid replacements 53E and 57E on the basis of the sequence of SEQ ID NO: 58, SEQ ID NO: 60, SEQ ID NO: 61, SEQ ID NO: 62, or SEQ ID NO: 63; amino acid replacements 52S, 53E, and 57E on the basis of the sequence of SEQ ID NO: 58, SEQ ID NO: 60, SEQ ID NO: 61, SEQ ID NO: 62, or SEQ ID NO: 63; amino acid replacements 49T and 82A on the basis of the sequence of SEQ ID NO: 58, SEQ ID NO: 60, SEQ ID NO: 61, SEQ ID NO: 62, or SEQ ID NO: 63; amino acid replacements 49T and 83Y on the basis of the sequence of SEQ ID NO: 58, SEQ ID NO: 60, SEQ ID NO: 61, SEQ ID NO: 62, or SEQ ID NO: 63; amino acid replacements 49T, 82A, and 83Y on the basis of the sequence of SEQ ID NO: 58, SEQ ID NO: 60, SEQ ID NO: 61, SEQ ID NO: 62, or SEQ ID NO: 63; or amino acid replacements 49T, 53E, 57E, and 82A on the basis of the sequence of SEQ ID NO: 58, SEQ ID NO: 60, SEQ ID NO: 61, SEQ ID NO: 62, or SEQ ID NO: 63. The positions for the amino acid replacements described above are amino acid residue positions numbered in natural order relative to the sequence SEQ ID NO: 58.

In some embodiments of the anti-IL23 antibody fusion protein according to any one of the above, the sequence of the TACI polypeptide is set forth in any one of SEQ ID NOs: 60-63 and SEQ ID NOs: 66-83. In some embodiments of the anti-IL23 antibody fusion protein according to any one of the above, the sequence of the TACI polypeptide is set forth in SEQ ID NO: 83.

In some embodiments, the anti-IL23 antibody fusion protein according to any one of the above comprises:
a first chain comprising [a TACI polypeptide 1]-[a linker 1]-[the heavy chain of the anti-IL23 antibody]-[a linker 2]-[a TACI polypeptide 2] from the N-terminus to the C-terminus, and
a second chain, being the light chain of the anti-IL23 antibody, wherein
the TACI polypeptide 1 and the TACI polypeptide 2 may be identical or different, and the TACI polypeptide 1 and the TACI polypeptide 2 may be independently selected from any TACI polypeptide described above; the linker 1 and the linker 2 may be identical or different; in some embodiments, the TACI polypeptide 1 and the TACI polypeptide 2 are identical; in some embodiments, the linker is a linker selected from the group consisting of (GₓS)_{y}, wherein x is selected from the group consisting of integers from 1 to 5 and y is selected from the group consisting of integers from 0 to 6; in some embodiments, the linker is GGGGSGGGGSGGGGS (set forth in SEQ ID NO: 84) or GGGS (set forth in SEQ ID NO: 85).

In some embodiments of the anti-IL23 antibody fusion protein according to any one of the above, in the anti-IL23 antibody fusion protein, the first chain is set forth in SEQ ID NO: 47 and the second chain is set forth in SEQ ID NO: 43, or the first chain is set forth in SEQ ID NO: 46 and the second chain is set forth in SEQ ID NO: 41. In some embodiments, the anti-IL23 antibody fusion protein according to any one of the above has two first chains set forth in SEQ ID NO: 47 and two second chains set forth in SEQ ID NO: 43; in some embodiments, the anti-IL23 antibody fusion protein has two first chains set forth in SEQ ID NO: 46 and two second chains set forth in SEQ ID NO: 41.

### Variants of Anti-IL23 Antibody or Fusion Protein Thereof

In certain embodiments, amino acid sequence variants of the anti-IL23 antibody or fusion protein thereof provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions, insertions, and/or substitutions of residues within the amino acid sequence of the anti-IL23 antibody or the fusion protein thereof. Any combination of deletion, insertion, and substitution can be made to obtain the final construct, provided that the final construct possesses the desired characteristics, e.g., antigen binding.

### Variants with Substitutions, Insertions, and Deletions

In certain embodiments, antibody variants having one or more amino acid substitutions are provided. Positions of interest for substitutional mutagenesis include CDRs and FRs. Conservative substitutions are shown in Table 3 under the heading of "Preferred substitution". More substantial changes are provided in Table 3 under the heading of "Exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

**Table 3. Amino acid substitutions**

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cvs(C) | Ser; Ala | Ser |
| Gln(Q) | Asn; Glu | Asn |
| Glu(E) | Asp; Gln | Asp |
| Glv(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. Particularly, HCDR3 and LCDR3 are often targeted.

In certain embodiments, substitutions, insertions or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In certain embodiments of the variant VH and VL sequences provided above, each CDR is unchanged or contains no more than 1, 2 or 3 amino acid substitutions.

A useful method for identifying residues or regions of an antibody that can be used as mutagenesis targets is called "alanine scanning mutagenesis". In this method, a residue or group of target residues (e.g., charged residues such as Arg, Asp, His, Lys and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme or a polypeptide that increases the serum half-life of the antibody.

### Modification of Fc Region

In one aspect, the Fc region of the anti-IL23 antibody or the anti-IL23 antibody fusion protein of the present disclosure comprises one or more amino acid substitutions that reduce its binding to an Fc receptor, e.g., its binding to the Fcy receptor, and reduce or eliminate effector functions. A natural IgG Fc region, specifically an IgG₁ Fc region or an IgG₄ Fc region, may cause the fusion protein of the present disclosure to target cells expressing an Fc receptor, rather than cells expressing an antigen. In some embodiments, the engineered Fc region of the present disclosure exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcyRI, FcyRIIa, FcyRIIB, or FcyRIIIa. In some embodiments, the engineered Fc region also has reduced binding affinity for a complement (e.g., C1q) as compared to the native Fc region. In some embodiments, the engineered Fc region has no reduced binding affinity for a neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG₁ Fc region, amino acid residue substitutions at positions such as positions 238, 265, 269, 270, 297, 327, and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions.

The anti-IL23 antibody or the anti-IL23 antibody fusion protein may comprise different antigen-binding domains fused to the two subunits of the Fc region and thus may result in undesired homodimerization. To improve yield and purity, it would be advantageous to introduce modifications that promote heterodimerization in the Fc region of the fusion protein of the present disclosure. In some embodiments, the Fc region of the present disclosure comprises modifications according to the knob-into-hole (KIH) technique, which involves introducing a protuberance structure (knob) at the interface of the first subunit and a pore structure (hole) at the interface of the second subunit. As such, the protuberance structure can be positioned in the pore structure, thereby promoting the formation of heterodimers and inhibiting the production of homodimers. The protuberance structure is constructed by substituting a small amino acid side chain at the interface of the first subunit with a larger side chain (e.g., tyrosine or tryptophan). The pore structure is created at the interface of the second subunit by substituting a large amino acid side chain with a smaller amino acid side chain (e.g., alanine or threonine). The protuberance and pore structures are prepared by altering the nucleic acid encoding the polypeptide, with optional amino acid substitutions shown in Table 4 below:

**Table 4. Combinations of KIH mutations**

| | | | | | | | | | |
|---|---|---|---|---|---|---|---|---|---|
| First subunit | T366Y | T366W | T394W | F405W | T366W | | | | 354C |
| | | | | | | T366Y | T366W | F405W | 356E |
| | | | | | | F405A | F405W | Y407A | 358M |
| | | | | | | | | | 366W |
| Second subunit | Y407T | Y407A | F405A | T394S | | | | | 349C |
| | | | | | T366S | | | | 356E |
| | | | | | L368A | T394W | T394W | T366W | 358M |
| | | | | | Y407V | Y407T | Y407A | T394S | 366S |
| | | | | | | | | | 368A |
| | | | | | | | | | 407V |

In addition to the knob-into-hole technique, other techniques for modifying the CH3 domain of the heavy chain of multispecific antibodies to achieve heterodimerization are also known in the art, e.g., WO 96/27011, WO 98/050431, EP 1870459, WO 2007/110205, WO 007/147901, WO 2009/089004, WO 2010/129304, WO 2011/90754, WO 2011/143545, WO 2012/058768, WO 2013/157954, and WO 013/096291.

The C-terminus of the Fc region may be an intact C-terminus ending with amino acid residues PGK or a shortened C-terminus, e.g., the shortened C-terminus in which one or two C-terminal amino acid residues have been removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

### Recombination Method

An anti-IL23 antibody or an anti-IL23 antibody fusion protein can be produced using recombination methods. For these methods, one or more isolated nucleic acids encoding the polypeptide or fusion protein are provided.

In one embodiment, the present disclosure provides an isolated nucleic acid encoding the anti-IL23 antibody or the anti-IL23 antibody fusion protein described above. Such nucleic acids may each independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide or fusion protein, wherein the method comprises culturing a host cell comprising a nucleic acid encoding the polypeptide or fusion protein, as provided above, under conditions suitable for expression, and optionally recovering the anti-IL23 antibody or the anti-IL23 antibody fusion protein from the host cell (or a host cell medium).

For recombinant production of the anti-IL23 antibody or the anti-IL23 antibody fusion protein, the nucleic acid encoding the protein is isolated and inserted into one or more vectors for further cloning and/or expression in a host cell. Such nucleic acids can be readily isolated and sequenced using conventional procedures, or produced by recombination methods or obtained by chemical synthesis.

Suitable host cells for cloning or expressing a vector encoding the anti-IL23 antibody or the anti-IL23 antibody fusion protein include prokaryotic or eukaryotic cells described herein. For example, the anti-IL23 antibody or the anti-IL23 antibody fusion protein can be produced in bacteria, particularly when glycosylation and Fc effector functions are not needed. After expression, anti-IL23 antibody or the anti-IL23 antibody fusion protein can be isolated from the bacterial cell paste in a soluble fraction and can be further purified. In addition to prokaryotes, eukaryotic microorganisms such as filamentous fungi or yeast are suitable cloning or expression hosts for vectors encoding fusion proteins, including fungal and yeast strains. Suitable host cells for expression of the fusion protein may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US 5959177, US 6040498, US 6420548, US 7125978, and US 6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

### Assay

The anti-IL23 antibody or the anti-IL23 antibody fusion protein provided herein can be identified, screened, or characterized for their physical/chemical characteristics and/or biological activities by a variety of assays known in the art. In one aspect, the anti-IL23 antibody or the anti-IL23 antibody fusion protein of the present disclosure is tested for activity, e.g., by known methods such as ELISA, western blot, and the like.

### Treatment Method and Route of Administration

Any of the anti-IL23 antibodies or anti-IL23 antibody fusion proteins provided herein can be used in the treatment method. In yet another aspect, the present disclosure provides use of the anti-IL23 antibody or the anti-IL23 antibody fusion protein in the manufacture or preparation of a medicament. In some embodiments, the B cell disorder or the autoimmune disease is a disease or condition associated with IL23. In some embodiments, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of tumors, chronic leukocytic leukemia, multiple myeloma, non-Hodgkin's lymphoma, post-transplant lymphoproliferative disorder, and light chain gammopathy. In some embodiments, the autoimmune disease is systemic lupus erythematosus. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the anti-IL23 antibody or the anti-IL23 antibody fusion protein, e.g., for any of the above pharmaceutical uses or treatment methods. In one embodiment, the pharmaceutical composition comprises any of the polypeptides or fusion proteins provided herein and a pharmaceutically acceptable carrier. In another embodiment, the pharmaceutical composition further comprises at least one additional therapeutic agent.

The anti-IL23 antibody or the anti-IL23 antibody fusion protein of the present disclosure can be used alone or in combination with additional agents for the treatment. For example, the anti-IL23 antibody or the anti-IL23 antibody fusion protein of the present disclosure can be co-administered with at least one additional therapeutic agent.

The anti-IL23 antibody or the anti-IL23 antibody fusion protein of the present disclosure (and any additional therapeutic agents) may be administered by any suitable means, including parenteral, intrapulmonary, and intranasal administration, and if required by local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The anti-IL23 antibody or the anti-IL23 antibody fusion protein of the present disclosure will be formulated, administered, and applied in a manner consistent with Good Medical Practice. Factors considered in this context include the specific condition being treated, the specific mammal being treated, the clinical state of the individual patient, the cause of the condition, the site of delivery of the agent, the method of administration, the timing of administration, and other factors known to medical practitioners. The polypeptide or fusion protein may be formulated with or without one or more agents currently used to prevent or treat the condition. The effective amount of such additional agents depends on the amount present in the pharmaceutical composition, the type of condition or treatment, and other factors. These are generally used in the same dosages and routes of administration as described herein, or in about 1% to 99% of the dosages described herein, or in other dosages, and by any route empirically/clinically determined to be appropriate. For the prevention or treatment of diseases, the appropriate dosage of the anti-IL23 antibody or the anti-IL23 antibody fusion protein of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to a patient in one or a series of treatments.

### Article of Manufacture

In another aspect of the present disclosure, provided is an article of manufacture, which comprises materials useful for the treatment, prevention, and/or diagnosis of the above conditions. The article of manufacture comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the anti-IL23 antibody or the anti-IL23 antibody fusion protein of the present disclosure. The label or package insert indicates that the composition is used to treat the selected condition. Further, the article of manufacture may comprise: (a) a first container containing a composition, wherein the composition comprises the anti-IL23 antibody or the anti-IL23 antibody fusion protein of the present disclosure; and (b) a second container containing a composition, wherein the composition comprises other cytotoxic agents or additional therapeutic agents. The article of manufacture in this embodiment of the present disclosure may further comprise a package insert indicating that the composition may be used to treat a particular condition. Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes.

### Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: Preparation of Mouse Anti-Human IL-23 p19 Monoclonal Antibodies

SJL mice were immunized with human IL-23 protein (Sino Biological, CT048-H08H). After the mice were immunized for 3 times, blood was taken to measure the titer of the antibody in the serum, and the mice in which the antibody titer in serum was high and was reaching a plateau were selected for spleen cell fusion. The fused hybridoma cells were plated in a 96-well cell culture plate and cultured in an incubator at 37 °C and 5*%* CO₂. The cell culture supernatant was taken for detection by enzyme linked immunosorbent assay (ELISA). The selected positive clones were expanded, frozen for seed preservation, and subcloned two to three times until single cell clones were obtained. The selected hybridoma clones were subjected to serum-free cell culturing to further prepare and purify antibodies. The obtained hybridoma antibodies were subjected to ELISA assay for detecting the binding of the antibodies to human IL-23 protein and the blocking of the receptors by the antibodies (see Test Example 1 and Test Example 2 of the present disclosure), and hybridoma cell strains with good binding activity and blocking activity were selected.

Monoclonal hybridoma cell strains mAb29 and mAb38 were selected, and the sequences of the monoclonal antibodies were cloned. The cloning process was as follows: hybridoma cells in the logarithmic growth phase were harvested, and the RNA was extracted using Trizol (Invitrogen, Cat #15596-018) and reverse-transcribed into cDNA. After PCR amplification using cDNA as a template, the cDNA was sequenced by a sequencing company, and the amino acid sequences of the variable regions of the antibodies corresponding to the obtained DNA sequences are shown below:
Amino acid sequence of mAb29 heavy chain variable region: 
QVQLKESGPGLVAPSQSLSITCTVSGFSLTSYAISWVRQPPGKGLEWLGVIWTGG
GTNYNSGIKSRLTISKDNSKSQVFLNMNSLQTEDTARYYCVREGDYGSYAGAM
DYWGQGTSVTVSS
SEQ ID NO: 1
Amino acid sequence of mAb29 light chain variable region: 
DIVLTQSPASLAVSLGQRATISCRASESVDYYGISSMHWFQQKPGQPPKLLIYRAS
NLESGIPARFSGSGSRTDFTLTINRVETDDVATYYCQQANKDPYTFGGGTKLEIK
SEQ ID NO: 2
Amino acid sequence of mAb38 heavy chain variable region: 
EFQLQQSGPELVKPGASVKISCKASGYSFTDHNMNWVKQSNGKSLEWIGVINP
NYGTTTYNQKFKGKATLTVDQSSSTAYMQLNILTSEDSAVYYCARRGFAYWGQ
GTLVTVSA
SEQ ID NO: 3
Amino acid sequence of mAb38 light chain variable region:

DIVISQDELSNPVTSGESVSISCRSSKSLLYKDGKTYLNWFLQRPGQSPQLLIYLM
STRASGVSDRFSGSGSGTDLTLEISRVKAEDVGVYYCQQIAEYPFTFGSGTKLEI
K
SEQ ID NO: 4

**Table 5. Antibody CDR sequences**

| Antibody | Heavy chain variable region | | Light chain variable region | |
|---|---|---|---|---|
| mAb29 | HCDR1 | SYAIS | LCDR1 | RASESVDYYGISSMH |
| | | SEQ ID NO: 5 | | SEQ ID NO: 8 |
| | HCDR2 | VIWTGGGTNYNSGIKS | LCDR2 | RASNLES |
| | | SEQ ID NO: 6 | | SEQ ID NO: 9 |
| | HCDR3 | EGDYGSYAGAMDY | LCDR3 | QQANKDPYT |
| | | SEQ ID NO: 7 | | SEQ ID NO: 10 |
| mAb38 | HCDR1 | DHNMN | LCDR1 | RSSKSLLYKDGKTYLN |
| | | SEQ ID NO: 11 | | SEQ ID NO: 14 |
| | HCDR2 | VINPNYGTTTYNQKFKG | LCDR2 | LMSTRAS |
| | | SEQ ID NO: 12 | | SEQ ID NO: 15 |
| | HCDR3 | RGFAY | LCDR3 | QQIAEYPFT |
| | | SEQ ID NO: 13 | | SEQ ID NO: 16 |

| | | | | |
|---|---|---|---|---|
| Note: the CDRs in the table are determined according to the Kabat numbering scheme. | | | | |

The variable region sequences of the candidate molecules mAb29 and mAb38 were PCR amplified for VH/VK sequences, respectively, which were subjected to homologous recombination with the expression vector pHr (with signal peptide and hIgG1/hkappa constant region genes (CH1-Fc/CL) fragment). Exemplarily, the human heavy chain IgG1 constant region sequence is set forth in SEQ ID NO: 38, the human light chain κ constant region sequence is set forth in SEQ ID NO: 39, a recombinant chimeric antibody full-length expression plasmid VH-CH1-Fc-pHr/VL-CL-pHr was constructed, and thereby chimeric antibodies Ch29 and Ch38 were obtained.

### Example 2: Humanization of Murine Anti-Human IL-23 Monoclonal Antibodies

By comparing with the IMGT human antibody heavy and light chain variable region germline gene database, heavy and light chain variable region germline genes with high homology were selected as templates, and CDRs of the murine antibody were grafted into corresponding humanized templates to form variable region sequences in the following order: FR1-CDR1-FR2-CDR2-FR3-CDR3-FR4, and then the variable region sequences were fused with human constant region sequences to obtain the humanized antibody. Humanization of the murine antibodies mAb29 and mAb38 is described below exemplarily, in which the CDR amino acid residues of the antibodies in the examples are determined and annotated by the Kabat numbering scheme.

### 1. Humanization of murine antibody mAb29

Heavy and light chain variable region germline genes with high homology were selected as templates. IGKV4-1*01 and IGKJ4*01 were selected as the template of the humanized light chain of the murine antibody mAb29, that is, FR1, FR2, and FR3 of the human germline light chain IGKV4-1*01 and JK4 region (as FR4) of IGKJ4*01 were selected as the humanized antibody light chain framework regions; IGHV2-26*01 and IGHJ6*01 were selected as the template of the humanized heavy chain, that is, FR1, FR2, and FR3 of the human germline heavy chain IGHV2-26*01 and JH6 region (as FR4) of IGHJ6*01 were selected as the humanized antibody light chain framework regions. Firstly, CDRs of the murine antibody mAb29 were grafted into selected corresponding humanization templates to replace the CDR regions of the humanization templates; then, in the humanized antibody, the amino acid residues at positions 4, 17, 36, 58, 60, and/or 68 (numbering according to Kabat numbering scheme) of the light chain variable region and the amino acid residues at positions 1, 30, 37, 44, 49, 73, 89, and/or 93 (numbering according to Kabat numbering scheme) of the heavy chain variable region were mutated. In addition, the amino acid residue at position 1 of the HCDR1: SYAIS (SEQ ID NO: 5) of the heavy chain variable region was mutated to N or Q, resulting in a novel HCDR1: NYAIS (SEQ ID NO: 27) or QYAIS (SEQ ID NO: 28). The variable region sequences of the humanized antibody of mAb29 are as follows:
> Amino acid sequence of hAb29VH1 (Graft + Q1E, A49G, A93V) 
EVTLKESGPVLVKPTETLTLTCTVSGFSLSSYAISWIRQPPGKALEWLGVIWTGG
GTNYNSGIKSRLTISKDTSKSQVVLTMTNMDPVDTATYYCVREGDYGSYAGAM
DYWGQGTTVTVSS
SEQ ID NO: 17
> Amino acid sequence of hAb29VH2 (Graft + Q1E, S30T, A49G, T73N, A93V) 
EVTLKESGPVLVKPTETLTLTCTVSGFSLTSYAISWIRQPPGKALEWLGVIWTGG
GTNYNSGIKSRLTISKDNSKSQVVLTMTNMDPVDTATYYCVREGDYGSYAGAM
DYWGQGTTVTVSS
SEQ ID NO: 18
> Amino acid sequence of hAb29VH3 (Graft + Q1E, S30T, I37V, A49G, T73N, T89R, A93V) 
EVTLKESGPVLVKPTETLTLTCTVSGFSLTSYAISWVRQPPGKALEWLGVIWTGG
GTNYNSGIKSRLTISKDNSKSQVVLTMTNMDPVDTARYYCVREGDYGSYAGAM
DYWGQGTTVTVSS
SEQ ID NO: 19
> Amino acid sequence of hAb29VH4 (Graft + S30T, I37V, A44G, A49G, T73N, T89R, A93V) 
QVTLKESGPVLVKPTETLTLTCTVSGFSLTSYAISWVRQPPGKGLEWLGVIWTGG
GTNYNSGIKSRLTISKDNSKSQVVLTMTNMDPVDTARYYCVREGDYGSYAGAM
DYWGQGTTVTVSS
SEQ ID NO: 20
> Amino acid sequence of hAb29VH5 (Graft + S30T, I37V, A44G, A49G, T73N, T89R, A93V + S31N) QVTLKESGPVLVKPTETLTLTCTVSGFSLTNYAISWVRQPPGKGLEWLGVIWTG 
GGTNYNSGIKSRLTISKDNSKSQVVLTMTNMDPVDTARYYCVREGDYGSYAGA
MDYWGQGTTVTVSS
SEQ ID NO: 21
> Amino acid sequence of hAb29VH6 (Graft + S30T, I37V, A44G, A49G, T73N, T89R, A93V + S31Q) 
QVTLKESGPVLVKPTETLTLTCTVSGFSLTQYAISWVRQPPGKGLEWLGVIWTG
GGTNYNSGIKSRLTISKDNSKSQVVLTMTNMDPVDTARYYCVREGDYGSYAGA
MDYWGQGTTVTVSS
SEQ ID NO: 22
> Amino acid sequence of hAb29VL1 (Graft + Y36F, G68R) 
DIVMTQSPDSLAVSLGERATINCRASESVDYYGISSMHWFQQKPGQPPKLLIYRA
SNLESGVPDRFSGSGSRTDFTLTISSLQAEDVAVYYCQQANKDPYTFGGGTKVEI
K
SEQ ID NO: 23
> Amino acid sequence of hAb29VL2 (Graft + M4L, Y36F, V58I, G68R) 
DIVLTQSPDSLAVSLGERATINCRASESVDYYGISSMHWFQQKPGQPPKLLIYRA
SNLESGIPDRFSGSGSRTDFTLTISSLQAEDVAVYYCQQANKDPYTFGGGTKVEI
K
SEQ ID NO: 24
> Amino acid sequence of hAb29VL3 (Graft + M4L, Y36F, V58I, D60A, G68R) 
DIVLTQSPDSLAVSLGERATINCRASESVDYYGISSMHWFQQKPGQPPKLLIYRA
SNLESGIPARFSGSGSRTDFTLTISSLQAEDVAVYYCQQANKDPYTFGGGTKVEIK
SEQ ID NO: 25
> Amino acid sequence of hAb29VL4 (Graft + M4L, E17Q, Y36F, V58I, D60A, G68R) 
DIVLTQSPDSLAVSLGQRATINCRASESVDYYGISSMHWFQQKPGQPPKLLIYRA
SNLESGIPARFSGSGSRTDFTLTISSLQAEDVAVYYCQQANKDPYTFGGGTKVEIK
SEQ ID NO: 26
Note: the sequence order is FR1-CDR1-FR2-CDR2-FR3-CDR3-FR4; in the sequences, the underlined part indicates a CDR region (CDR amino acid residues are determined by Kabat numbering scheme), and the remainder indicates the FR region.

### 2. Humanization of murine antibody mAb38

IGKV2-40*01 and IGKJ2*01 were selected as the template of the humanized light chain of the murine antibody mAb38, that is, FR1, FR2, and FR3 of the human germline light chain IGKV2-40*01 and JK2 region (as FR4) of IGKJ2*01 were selected as the humanized antibody light chain framework regions; IGHV1-3*01 and IGHJ1*01 were selected as the template of the humanized heavy chain, that is, FR1, FR2, and FR3 of the human germline heavy chain IGHV1-3*01 and JH1 region (as FR4) of IGHJ1*01 were selected as the humanized antibody heavy chain framework regions. Firstly, CDRs of the murine antibody mAb38 were grafted into selected corresponding humanization templates to replace the CDR regions of the humanization templates; then, in the humanized antibody, the amino acid residues at positions 4, 36, 39, 71, and/or 100 (numbering according to Kabat numbering scheme) of the light chain variable region and the amino acid residues at positions 1, 2, 28, 44, 71, and/or 73 (numbering according to Kabat numbering scheme) of the heavy chain variable region were mutated. In addition, the amino acid residue at position 3 of the LCDR2: LMSTRAS (SEQ ID NO: 15) of the light chain variable region was mutated to Q, resulting in a novel LCDR2: LMQTRAS (SEQ ID NO: 37). The variable region sequences of the humanized antibody of mAb38 are as follows:
> Amino acid sequence of hAb38VHl (Graft + Q1E, V2F, R71V) 
EFQLVQSGAEVKKPGASVKVSCKASGYTFTDHNMNWVRQAPGQRLEWMGVI
NPNYGTTTYNQKFKGRVTITVDTSASTAYMELSSLRSEDTAVYYCARRGFAYWG
QGTLVTVSS
SEQ ID NO: 29
> Amino acid sequence of hAb38VH2 (Graft + Q1E, T28S, R71V) 
EVQLVQSGAEVKKPGASVKVSCKASGYSFTDHNMNWVRQAPGQRLEWMGVI
NPNYGTTTYNQKFKGRVTITVDTSASTAYMELSSLRSEDTAVYYCARRGFAYWG
QGTLVTVSS
SEQ ID NO: 30
> Amino acid sequence of hAb38VH3 (Graft + Q1E, V2F, T28S, R71V, T73Q) 
EFQLVQSGAEVKKPGASVKVSCKASGYSFTDHNMNWVRQAPGQRLEWMGVI
NPNYGTTTYNQKFKGRVTITVDQSASTAYMELSSLRSEDTAVYYCARRGFAYW
GQGTLVTVSS
SEQ ID NO: 31
> Amino acid sequence of hAb38VH4 (Graft + Q1E, V2F, T28S, R44S, R71V, T73Q) 
EFQLVQSGAEVKKPGASVKVSCKASGYSFTDHNMNWVRQAPGQSLEWMGVIN
PNYGTTTYNQKFKGRVTITVDQSASTAYMELSSLRSEDTAVYYCARRGFAYWG
QGTLVTVSS
SEQ ID NO: 32
> Amino acid sequence of hAb38VL1 (Graft + Q100S) 
DIVMTQTPLSLPVTPGEPASISCRSSKSLLYKDGKTYLNWYLQKPGQSPQLLIYL
MSTRASGVPDRFSGSGSGTDFTLKISRVEAEDVGVYYCQQIAEYPFTFGSGTKLE
IK
SEQ ID NO: 33
> Amino acid sequence of hAb38VL2 (Graft + Y36F, F71L, Q100S) 
DIVMTQTPLSLPVTPGEPASISCRSSKSLLYKDGKTYLNWFLQKPGQSPQLLIYL
MSTRASGVPDRFSGSGSGTDLTLKISRVEAEDVGVYYCQQIAEYPFTFGSGTKLE
IK
SEQ ID NO: 34
> Amino acid sequence of hAb38VL3 (Graft + M4I, Y36F, K39R, F71L, Q100S) 
DIVITQTPLSLPVTPGEPASISCRSSKSLLYKDGKTYLNWFLQRPGQSPQLLIYLM
STRASGVPDRFSGSGSGTDLTLKISRVEAEDVGVYYCQQIAEYPFTFGSGTKLEI
K
 SEQ ID NO: 35
> Amino acid sequence of hAb38VL4 (Graft + Y36F, F71L, Q100S + S52Q) 
DIVMTQTPLSLPVTPGEPASISCRSSKSLLYKDGKTYLNWFLQKPGQSPQLLIYL
MQTRASGVPDRFSGSGSGTDLTLKISRVEAEDVGVYYCQQIAEYPFTFGSGTKL
EIK
SEQ ID NO: 36 Note: the sequence order is FR1-CDR1-FR2-CDR2-FR3-CDR3-FR4; in the sequences, the underlined part indicates a CDR region (CDR amino acid residues are determined by Kabat numbering scheme), and the remainder indicates the FR region.

### 3. Preparation of humanized antibodies

Expression vectors of the light chain and the heavy chain of an antibody were constructed; cross-pairing combination was performed on the light chain and the heavy chain of a humanized antibody, and the culture supernatant was collected and purified after 293E cells were transfected to obtain the humanized full-length antibody. The humanized antibody heavy chain constant region could be selected from the group consisting of IgG1, IgG2, IgG3, and IgG4 constant regions; Exemplarily, human heavy chain IgG1 constant region was used, and the humanized antibody light chain constant region could be selected from the group consisting of human κ and λ chain constant regions. Exemplary antibody constant region sequences are as follows:
Amino acid sequence of human IgG1 heavy chain constant region (containing mutations L234A and L235A) 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 38
> Amino acid sequence of human κ light chain constant region



SEQ ID NO: 39

Exemplarily, the heavy chain variable region of the humanized antibody of mAb29 described above was fused to the human heavy chain IgG1 constant region (with the sequence set forth in SEQ ID NO: 38) to form an antibody full-length heavy chain, and the light chain variable region of the humanized antibody was fused to the human κ light chain constant region (with the sequence set forth in SEQ ID NO: 39) to form an antibody full-length light chain, resulting in the humanized antibodies of mAb29 shown in Table 6 below:

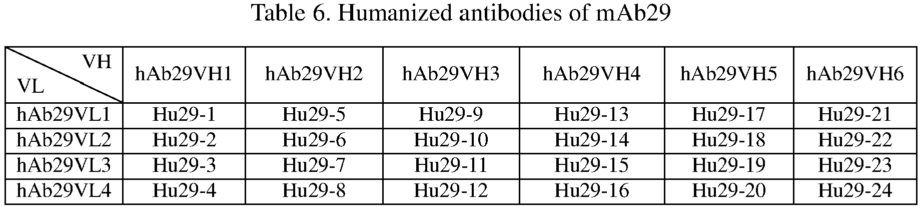

In addition, the heavy chain variable region of the humanized antibody of mAb38 described above was fused to the human heavy chain IgG1 constant region (with the sequence set forth in SEQ ID NO: 38) to form an antibody full-length heavy chain, and the light chain variable region of the humanized antibody was fused to the human κ light chain constant region (with the sequence set forth in SEQ ID NO: 39) to form an antibody full-length light chain, resulting in the humanized antibodies of mAb38 shown in Table 7 below:

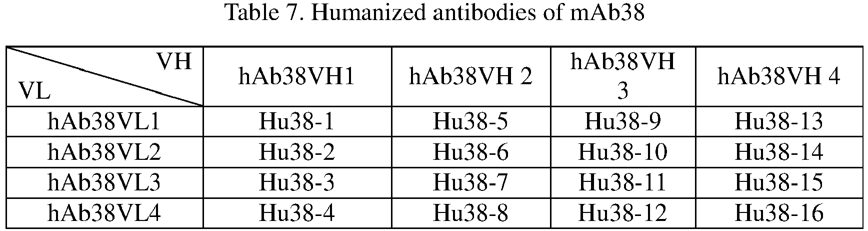

Exemplarily, the light/heavy chain full-length sequences of the humanized antibodies are shown in Table 8 below:

**Table 8. Light/heavy chain sequences of humanized antibodies**

| Antibody No. | Heavy chain sequence | Light chain sequence |
|---|---|---|
| Hu29-19 | QVTLKESGPVLVKPTETLTLTCTVSGFSLTN YAISWVRQPPGKGLEWLGVIWTGGGTNYN SGIKSRLTISKDNSKSQVVLTMTNMDPVDT ARYYCVREGDYGSYAGAMDYWGQGTTVT VSSASTKGPSVFPLAPSSKSTSGGTAALGCLVK DYFPEPVTVSWNSGALTSGVHTFPAVLQSSGL YSLSSVVTVPSSSLGTQTYICNVNHKPSNTKVD KKVEPKSCDKTHTCPPCPAPEAAGGPSVFLF PPKPKDTLMISRTPEVTCVVVDVSHEDPEVKF NWYVDGVEVHNAKTKPREEQYNSTYRVVSVL TVLHQDWLNGKEYKCKVSNKALPAPIEKTISK AKGQPREPQVYTLPPSRDELTKNQVSLTCLVK GFYPSDIAVEWESNGQPENNYKTTPPVLDSD GSFFLYSKLTVDKSRWQQGNVFSCSVMHEAL HNHYTQKSLSLSPGK (SEQ ID NO: 40) | DIVLTQSPDSLAVSLG ERATINCRASESVDYY GISSMHWFQQKPGQP PKLLIYRASNLESGIPA RFSGSGSRTDFTLTISS LQAEDVAVYYCQQAN KDPYTFGGGTKVEIK RTVAAPSVFIFPPSDEQ LKSGTASVVCLLNNFYP REAKVQWKVDNALQS GNSQESVTEQDSKDST YSLSSTLTLSKADYEKH KVYACEVTHQGLSSPVT KSFNRGEC (SEQ ID NO: 41) |
| Hu29-24 | QVTLKESGPVLVKPTETLTLTCTVSGFSLTQ YAISWVRQPPGKGLEWLGVIWTGGGTNYN SGIKSRLTISKDNSKSQVVLTMTNMDPVDT ARYYCVREGDYGSYAGAMDYWGQGTTVT VSSASTKGPSVFPLAPSSKSTSGGTAALGCLVK DYFPEPVTVSWNSGALTSGVHTFPAVLQSSGL YSLSSVVTVPSSSLGTQTYICNVNHKPSNTKVD KKVEPKSCDKTHTCPPCPAPEAAGGPSVFLF PPKPKDTLMISRTPEVTCVVVDVSHEDPEVKF NWYVDGVEVHNAKTKPREEQYNSTYRVVSVL TVLHQDWLNGKEYKCKVSNKALPAPIEKTISK AKGQPREPQVYTLPPSRDELTKNQVSLTCLVK GFYPSDIAVEWESNGQPENNYKTTPPVLDSD GSFFLYSKLTVDKSRWQQGNVFSCSVMHEAL HNHYTQKSLSLSPGK (SEQ ID NO: 42) | DIVLTQSPDSLAVSLG QRATINCRASESVDY YGISSMHWFQQKPGQ PPKLLIYRASNLESGIP ARFSGSGSRTDFTLTIS SLQAEDVAVYYCQQA NKDPYTFGGGTKVEI KRTVAAPSVFIFPPSDE QLKSGTASVVCLLNNFY PREAKVQWKVDNALQ SGNSQESVTEQDSKDS TYSLSSTLTLSKADYEK HKVYACEVTHQGLSSP VTKSFNRGEC (SEQ ID NO: 43) |
| Hu38-4 | EFQLVQSGAEVKKPGASVKVSCKASGYTF TDHNMNWVRQAPGQRLEWMGVINPNYGT TTYNQKFKGRVTITVDTSASTAYMELSSLR SEDTAVYYCARRGFAYWGQGTLVTVSSAST KGPSVFPLAPSSKSTSGGTAALGCLVKDYFPE PVTVSWNSGALTSGVHTFPAVLQSSGLYSLSSV VTVPSSSLGTQTYICNVNHKPSNTKVDKKVEP KSCDKTHTCPPCPAPEAAGGPSVFLFPPKPK DTLMISRTPEVTCVVVDVSHEDPEVKFNWYV DGVEVHNAKTKPREEQYNSTYRVVSVLTVLH QDWLNGKEYKCKVSNKALPAPIEKTISKAKGQ PREPQVYTLPPSRDELTKNQVSLTCLVKGFYP SDIAVEWESNGQPENNYKTTPPVLDSDGSFFL YSKLTVDKSRWQQGNVFSCSVMHEALHNHYT QKSLSLSPGK (SEQ ID NO: 44) | DIVMTQTPLSLPVTPG EPASISCRSSKSLLYKD GKTYLNWFLQKPGQS PQLLIYLMQTRASGV PDRFSGSGSGTDLTLK ISRVEAEDVGVYYCQ QIAEYPFTFGSGTKLEI KRTVAAPSVFIFPPSDE QLKSGTASVVCLLNNFY PREAKVQWKVDNALQ SGNSQESVTEQDSKDS TYSLSSTLTLSKADYEK HKVYACEVTHQGLSSP VTKSFNRGEC (SEQ ID NO: 45) |

### Example 3: Construction of Anti-IL23 Antibody Fusion Proteins

The anti-IL23 antibodies described above were fused to a TACI polypeptide to construct anti-IL23 antibody fusion proteins. Exemplarily, the anti-IL23 antibody is Hu29-19 or Hu29-24. The TACI polypeptide may be any suitable TACI polypeptide. Exemplarily, the TACI polypeptide is the TACI polypeptide described in Chinese patent application 202110348497.6 (entitled "novel TACI polypeptide, fusion protein and uses thereof' filed on March 31, 2021) and patent applications that claim priority thereto (incorporated herein by reference in its entirety), such as the TACI polypeptides shown in Table 9 below:

**Table 9. Amino acid sequences of TACI polypeptides**

| Polypeptide No. | SEQ ID NO: | Amino acid sequence |
|---|---|---|
| TACI-0 | 51 | MSGLGRSRRGGRSRVDQEERFPQGLWTGVAMRSCP EEQYWDPLLGTCMSCKTICNHQSQRTCAAFCRSLSC RKEQGKFYDHLLRDCISCASICGQHPKQCAYFCENK |
| | | LRSPVNLPPELRRQRSGEVENNSDNSGRYQGLEHRG SEASPALPGLKLSADQVALVYS |
| TACI-1 | 52 | SGLGRSRKGGKSRVDQEERFPQGLWTGVAMRSCPEE QYWDPLLGTCMSCKTICNHQSQRTCAAFCRSLSCRK EQGKFYDHLLRDCISCASICGQHPKQCAYFCENKLRS PVNLPPELRKQRSGEVEQNSDNSGKYQGLEHRGSEA SPALPGLKLSADQVALVYS |
| TACI-2 | 53 | SGLGRSRKGGKSRVDQEERFPQGLWTGVAMRSCPEE QYWDPLLGTCMSCKTICNHQSQRTCAAFCRSLSCRK EQGKFYDHLLRDCISCASICGQHPKQCAYFCENKLRS PVNLPPELRKQRSGEVENNSDNSG |
| TACI-3 | 54 | SGLGRSRKGGKSRVDQEERFPQGLWTGVAMRSCPEE QYWDPLLGTCMSCKTICNHQSQRTCAAFCRSLSCRK EQGKFYDHLLRDCISCASICGQHPKQCAYFCENKLRS PVNLPPELRKQRSGEVE |
| TACI-4 | 55 | GGRSRVDQEERFPQGLWTGVAMRSCPEEQYWDPLL GTCMSCKTICNHQSQRTCAAFCRSLSCRKEQGKFYD HLLRDCISCASICGQHPKQCAYFCENKLRSPVNLPPE LRKQRSGEVENNSDNSGKYQGLEHRGSEASPALPGL KLSADQVALVYS |
| TACI-5 | 56 | GGRSRVDQEERFPQGLWTGVAMRSCPEEQYWDPLL GTCMSCKTICNHQSQRTCAAFCRSLSCRKEQGKFYD HLLRDCISCASICGQHPKQCAYFCENKLRSPVNLPPE LRKQRSGEVENNSDNSG |
| TACI-6 | 57 | GGRSRVDQEERFPQGLWTGVAMRSCPEEQYWDPLL GTCMSCKTICNHQSQRTCAAFCRSLSCRKEQGKFYD HLLRDCISCASICGQHPKQCAYFCENKLRSPVNLPPE LRKQRSGEVE |
| TACI-7 | 58 | FPQGLWTGVAMRSCPEEQYWDPLLGTCMSCKTICN HQSQRTCAAFCRSLSCRKEQGKFYDHLLRDCISCASI CGQHPKQCAYFCENKLRSPVNLPP |
| TACI-8 | 59 | SLSCRKEQGKFYDHLLRDCISCASICGQHPKQCAYFC ENKLRSPVNLPPELRKQRSGEVENNSDNSGKYQGLE HRGSEASPALPGLKLSADQVALVYS |
| TACI-9 | 60 | SLSCRKEQGKFYDHLLRDCISCASICGQHPKQCAYFC ENKL |
| TACI-10 | 61 | SLSCRKEQGKFYDHLLRDCISCASICGQHPKQCAYFC ENK |
| TACI-11 | 62 | SLSCRKEQGKFYDHLLRDCISCASICGQHPKQCAYFC EN |
| TACI-12 | 63 | SLSCRKEQGKFYDHLLRDCISCASICGQHPKQCAYFC E |
| TACI-13 | 64 | SLSCRKEQGKFYDHLLRDCISCASICGQHPKQCAYFC |
| TACI-14 | 65 | SLSCRKEQGKFYDHLLRDCISCASICGQHPKQCAYF |
| TACI-9-1 | 66 | STSCRKEQGKFYDHLLRDCISCASICGQHPKQCAYFC ENKL |
| TACI-9-2 | 67 | SLSCSKEQGKFYDHLLRDCISCASICGQHPKQCAYFC ENKL |
| TACI-9-3 | 68 | SLSCREEQGKFYDHLLRDCISCASICGQHPKQCAYFC ENKL |
| TACI-9-4 | 69 | SLSCRQEQGKFYDHLLRDCISCASICGQHPKQCAYFC ENKL |
| TACI-9-5 | 70 | SLSCRKEQGEFYDHLLRDCISCASICGQHPKQCAYFC ENKL |
| TACI-9-6 | 71 | SRSCRKEQGKFYDHLLRTCISCASICGQHPKQCAYFC ENKL |
| TACI-9-7 | 72 | SRSCRKEQGKFYDHLLRACISCASICGQHPKQCAYFC ENKL |
| TACI-9-8 | 73 | SLSCRKEQGKFYDHLLRDCISCASICGQHPKQCAAFC ENKL |
| TACI-9-9 | 74 | SRSCRKEQGKFYDHLLRTCISCASICGQHPKQCARFC ENKL |
| TACI-9-10 | 75 | SLSCREEQGEFYDHLLRDCISCASICGQHPKQCAYFC ENKL |
| TACI-9-11 | 76 | SLSCSEEQGEFYDHLLRDCISCASICGQHPKQCAYFC ENKL |
| TACI-9-12 | 77 | STSCRKEQGKFYDHLLRDCISCASICGQHPKQCAAFC ENKL |
| TACI-9-13 | 78 | STSCRKEQGKFYDHLLRDCISCASICGQHPKQCAYYC ENKL |
| TACI-9-14 | 79 | STSCRKEQGKFYDHLLRDCISCASICGQHPKQCAAY CENKL |
| TACI-9-15 | 80 | STSCREEQGEFYDHLLRDCISCASICGQHPKQCAAFC ENKL |
| TACI-9-15a | 81 | STSCREEQGEFYDHLLRDCISCASICGQHPKQCAAFC ENK |
| TACI-9-15b | 82 | STSCREEQGEFYDHLLRDCISCASICGQHPKQCAAFC EN |
| TACI-9-15c | 83 | STSCREEQGEFYDHLLRDCISCASICGQHPKQCAAFC E |

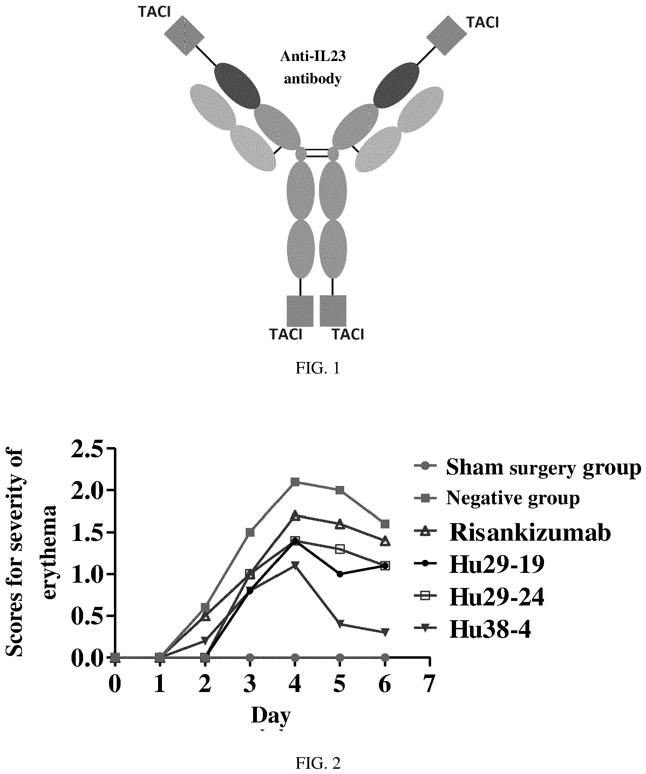

Exemplarily, two TACI polypeptides (e.g., TACI-9-15c) were fused to the N-terminus and the C-terminus, respectively, of the heavy chain of an anti-IL23 antibody (e.g., Hu29-19 or Hu29-24), and SS was added as a protective amino acid to the C-terminus of the TACI polypeptide fused to the C-terminus of the heavy chain of the antibody to prevent the TACI polypeptide from being cleaved by carboxypeptidase. The schematic structural diagram of the constructed anti-IL23 antibody fusion proteins (Hu29-19T and Hu29-24T) is shown in FIG. 1, and the amino acid sequences of the heavy and light chains are as follows:
> Amino acid sequence of Hu29-19T heavy chain (SEQ ID NO: 46) 
STSCREEQGEFYDHLLRDCISCASICGQHPKQCAAFCEGGGGSGGGGSGGGGS
QVTLKESGPVLVKPTETLTLTCTVSGFSLTNYAISWVRQPPGKGLEWLGVIWTG
GGTNYNSGIKSRLTISKDNSKSQVVLTMTNMDPVDTARYYCVREGDYGSYAGA
MDYWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
 
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
GGGSSTSCREEQGEFYDHLLRDCISCASICGQHPKQCAAFCESS
> Hu29-19T light chain (same as Hu29-19 light chain, SEQ ID NO: 41)
> Amino acid sequence of Hu29-24T heavy chain (SEQ ID NO: 47) 
STSCREEQGEFYDHLLRDCISCASICGQHPKQCAAFCEGGGGSGGGGSGGGGS
QVTLKESGPVLVKPTETLTLTCTVSGFSLTQYAISWVRQPPGKGLEWLGVIWTG
GGTNYNSGIKSRLTISKDNSKSQVVLTMTNMDPVDTARYYCVREGDYGSYAGA
MDYWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
GGGSSTSCREEQGEFYDHLLRDCISCASICGQHPKQCAAFCESS
> Hu29-24T light chain (same as Hu29-24 light chain, SEQ ID NO: 43)
Note: in the sequences, the single underlined part indicates a TACI sequence, the double-underlined part indicates an antibody variable region, the italic part indicates an antibody constant region, and the bold part indicates a linker.

The sequence of the control molecule risankizumab (see WHO Drug Information Vol. 30, No. 1, 2016) in the present disclosure is shown below:
> Amino acid sequence of risankizumab heavy chain (SEQ ID NO: 48) 
QVQLVQSGAEVKKPGSSVKVSCKASGYTFTDQTIHWMRQAPGQGLEWIGYIYP
RDDSPKYNENFKGKVTITADKSTSTAYMELSSLRSEDTAVYYCAIPDRSGYAWFI
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKSCDKTHTCPPCPAPEAAGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVM
HEALHNHYTQKSLSLSPG
> Amino acid sequence of risankizumab light chain (SEQ ID NO: 49)

DIQMTQSPSSLSASVGDRVTITCKASRDVAIAVAWYQQKPGKVPKLLIYWASTR
HTGVPSRFSGSGSRTDFTLTISSLQPEDVADYFCHQYSSYPFTFGSGTKLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

The sequence of the control molecule RCT-18 (tefitacicept, RC18, RemeGen, WHO (sequence No. 10932)) is shown below.
> Amino acid sequence of RCT-18 (SEQ ID NO: 50) 
SRVDQEERFPQGLWTGVAMRSCPEEQYWDPLLGTCMSCKTICNHQSQRTCAAF
CRSLSCRKEQGKFYDHLLRDCISCASICGQHPKQCAYFCENKLRSPVNLPPELDK
THTCPPCPAPEAEGAPSVFLFPPKPKDTLMISRTPEVTCVVVDVSHEDPEVKFNW
YVDGVEVHNAKTKPREEQYNSTYRVVSVLTVLHQDWLNGKEYKCKVSNKALP
SSIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQ
KSLSLSPGK
 Note: in the sequence, the underlined part indicates a TACI fragment, and the remainder indicates Fc.

### Test Examples

### Test Example 1: Elisa Binding Assay

The binding activity of the test molecules for IL-23 p19 and BAFF was measured by Elisa (coating the test molecules). The specific procedures are as follows:
The test sample was diluted to 2 µg/mL with a PBS (BasalMedia, B320) buffer at pH 7.4 and added to a 96-well microplate (Corning, 3590) at 100 µL/well. The plate was incubated at 4 °C overnight. After the liquid was discarded, 5% skim milk (BD, 232100) diluted with PBS was added at 300 µL/well for blocking, and the mixture was incubated at 37 °C for 2 h. After the blocking was completed, the blocking solution was discarded, and the plate was washed 3 times with a PBST buffer (pH 7.4, PBS containing 0.1% tween-20). The IL-23 (Sino Biological, CT048-H08H) or BAFF (ACROBiosystems, BAF-H52D4) solution diluted in a gradient was added at 100 µL/well, and the mixture was incubated at 37 °C for 1 h. After the incubation, the plate was washed 3 times with PBST. When the binding to IL-23 and BAFF was to be detected, Anti-His-HRP (Sino biological, 105327-MM02T-H, diluted at 1:2000) was added at 100 µL/well, and the mixture was incubated at 37 °C for 1 h. After the plate was washed 3 times with PBST, a TMB chromogenic substrate (KPL, 5120-0077) was added at 100 µL/well, and the mixture was incubated at room temperature for 10-15 min, and then 1 M H₂SO₄ was added at 50 µL/well to terminate the reaction. The absorbance values at 450 nm were taken using a microplate reader. The binding curves of antibodies to antigens were fitted using software, and the EC₅₀ values were calculated.

The experimental results of the binding of the anti-IL23 antibodies and the fusion proteins thereof to human IL-23 are shown in Table 10 below:

**Table 10: Experimental results of binding to IL-23 protein**

| Sample | Binding activity EC₅₀ for human IL-23 (nM) | Binding activity EC₅₀ for human BAFF (nM) |
|---|---|---|
| Ch29 | 1.155 | - |
| Ch38 | 1.831 | - |
| Hu29-15 | 1.189 | - |
| Hu29-19 | 1.107 | - |
| Hu29-24 | 1.218 | - |
| Hu38-4 | 1.876 | - |
| Hu29-19T | 1.425 | 5.211 |
| Hu29-24T | 1.525 | 5.638 |
| RCT-18 | | 21.49 |

Experimental results show that the anti-IL23 antibodies and the fusion proteins thereof of the present disclosure can specifically bind to human IL-23 protein, and the anti-IL23 antibodies and the fusion proteins thereof can also specifically bind to human BAFF protein, and the binding activity is stronger than that of the control RCT-18.

### Test Example 2: Experiment on Blocking of Ligands and Receptors

The blocking activity of the anti-IL23 antibodies and the fusion proteins thereof for receptors and ligands of IL-23/IL-23R, BAFF/BAFF-R, BAFF/BCMA, BAFF/TACI, APRIL/BCMA, APRIL/TACI and the like was detected by Elisa. The specific procedures are as follows:
The receptor protein was diluted to 2 µg/mL with a PBS (BasalMedia, B320) buffer at pH 7.4 and added to a 96-well microplate (Corning, 3590) at 100 µL/well. The plate was incubated at 4 °C overnight. After the liquid was discarded, 1% Casein blocking solution (Thermo, 37528) was added at 200 µL/well for blocking, and the plate was incubated at 37 °C for 2 h. After the blocking was complete, the blocking solution was discarded, and the plate was washed 3 times with a PBST buffer (pH 7.4 PBS containing 0.1% tween-20) for later use. After the biotin-labeled ligand protein at a fixed concentration was mixed with an antibody or fusion protein diluted in a gradient, the mixture was pre-incubated at 37 °C for 30 min and then added to the blocked microplate. The plate was incubated at 37 °C for 1.5 h. After the incubation was complete, the plate was washed 3 times with PBST. Streptavidin-HRP (Invitrogen, 434323, diluted at 1:4000) was added at 100 µL/well, and the plate was incubated at 37 °C for 1 h. The supernatant was removed. After the plate was washed 3 times with PBST, TMB chromogenic substrate (KPL, 5120-0077) was added at 100 µL/well, and the plate was incubated at room temperature for 10-15 min. 1 M H₂SO₄ was added at 50 µL/well to terminate the reaction. The absorbance values at 450 nm were taken using a microplate reader. Curves for the inhibition of binding of the ligands to the receptors were fitted using software, and the IC₅₀ values were calculated. The source information of the ligand proteins used in this test example is as follows: IL-23 (Sino Biological, CT048-H08H), BAFF (Sino Biological, 10056-HNCH), APRIL (R&D Systems, 5860-AP-010/CF). The source information of the receptor proteins used is as follows: IL-23R (Sino Biological, 13840-H02H), BAFF-R (Sino Biological, 16079-H02H), BCMA (Sino Biological, 10620-H02H), TACI (ACROBiosystems, TAI-H5256).

The experimental results of the blocking of IL-23/IL-23R by the anti-IL23 antibodies and the fusion proteins thereof are shown in Table 11 below:

**Table 11. Experimental results of blocking of IL-23/IL-23R by the anti-IL23 antibodies and the fusion proteins thereof**

| Sample | Blocking activity IC₅₀ for IL-23/IL-23R (nM) |
|---|---|
| Ch29 | 0.3858 |
| Ch38 | 0.3613 |
| Hu29-19 | 0.4783 |
| Hu29-24 | 0.4877 |
| Hu38-4 | 0.4274 |
| Hu29-19T | 0.4968 |
| Hu29-24T | 0.3137 |
| Risankizumab | 0.6104 |

The experimental results of the blocking of the binding of BAFF protein to receptors thereof by the anti-IL23 antibody fusion proteins are shown in Table 12 below:

**Table 12. Experimental results of blocking of BAFF protein by anti-IL23 antibody fusion proteins**

| Sample | Blocking activity for BAFF/BAFF-R (IC₅₀, nM) | Blocking activity for BAFF/BCMA (IC₅₀, nM) | Blocking activity for BAFF/TACI (IC₅₀, nM) |
|---|---|---|---|
| Hu29-19T | 0.8387 | 0.9927 | 1.273 |
| Hu29-24T | 1.031 | 0.9469 | 1.075 |
| RCT-18 | 7.570 | 4.857 | 6.072 |

The experimental results of the blocking of the binding of APRIL protein to receptors thereof by the anti-IL23 antibody fusion proteins are shown in Table 13 below:

**Table 13. Experimental results of blocking of APRIL protein by anti-IL23 antibody fusion proteins**

| Sample | Blocking activity for APRIL/BCMA (IC₅₀, nM) | Blocking activity for APRIL/TACI (IC₅₀, nM) |
|---|---|---|
| Hu29-19T | 0.3384 | 2.095 |
| Hu29-24T | 0.08270 | 1.389 |
| RCT-18 | 484.1 | 64.89 |

The experimental results of the blocking of IL-23/IL-23R by other anti-IL23 antibodies are shown in Table 14 below:

**Table 14. Experimental results of blocking of IL-23/IL-23R by anti-IL23 antibodies**

| Sample | Blocking activity IC₅₀ for IL-23/IL-23R (nM) |
|---|---|
| Hu29-1 | 0.4743 |
| Hu29-2 | 0.3972 |
| Hu29-5 | 0.3881 |
| Hu29-6 | 0.4367 |
| Hu29-9 | 0.3966 |
| Hu29-10 | 0.4242 |
| Hu29-15 | 0.4104 |
| Hu38-2 | 0.465 |
| Hu38-5 | 0.2279 |
| Hu38-9 | 0.4963 |
| Hu38-13 | 0.455 |

Experimental results show that the anti-IL23 antibodies or the anti-IL23 antibody fusion proteins constructed in the present disclosure can effectively block IL-23/IL-23R binding, and the activity of the anti-IL23 antibody fusion proteins for blocking BAFF and receptors thereof or APRIL and receptors thereof is significantly stronger than that of the control RCT-18.

### Test Example 3: Experiment on Proliferation of BaF3-IL-23R Cells

The *in vitro* activity of the antibodies and fusion proteins was detected by the experiment on proliferation of BaF3-IL-23R cells. The experimental procedures are as follows: BaF3 cells (Cobioer, CBP60474) stably expressing IL-23R and IL-12Rβ1 were plated in a 96-well cell plate (Corning, 3903) at 50 µL/well, i.e., 4000 cells. The test sample diluted in a gradient was mixed with IL-23 protein (Sino Biological, CT048-H08H) at a fixed concentration, and the mixture was added to the cell culture plate. The resulting mixture was cultured in an incubator at 37 °C for 72 h. The cell culture plate was then taken out, and the Celltiter Glo detection solution (Promega, G755B) was added at 50 µL/well. The plate was incubated in a shaker for 10 min and then taken out and left to stand at room temperature for 10 min, and bioluminescent signals were detected using a microplate reader (PerkinElmer, Victor3). Based on the data, inhibition curves were fitted using software, and IC₅₀ values were calculated.

The experimental results of the inhibition of proliferation of BaF3-IL-23R cells by anti-IL23 antibodies and fusion proteins thereof are shown in Table 15 below:

**Table 15. Experimental results of inhibition of proliferation of BaF3-IL-23R cells by anti-IL23 antibodies and fusion proteins thereof**

| Sample name | Activity in inhibiting proliferation of BaF3-IL-23R cells (IC₅₀, nM) |
|---|---|
| Ch29 | 0.1753 |
| Ch38 | 0.2221 |
| Hu29-19 | 0.1938 |
| Hu29-24 | 0.1784 |
| Hu29-19T | 0.1385 |
| Hu29-24T | 0.2316 |

The experimental results of the inhibition of proliferation of BaF3-IL-23R cells by other anti-IL23 antibodies are shown in Table 16 below:

**Table 16. Experimental results of inhibition of proliferation of BaF3-IL-23R cells by anti-IL23 antibodies**

| Sample | Activity in inhibiting proliferation of BaF3-IL-23R cells (IC₅₀, nM) |
|---|---|
| Hu38-1 | 0.1675 |
| Hu38-2 | 0.1384 |
| Hu38-3 | 0.1736 |
| Hu38-4 | 0.1219 |
| Hu38-5 | 0.1477 |
| Hu38-6 | 0.1358 |
| Hu38-7 | 0.1794 |
| Hu38-8 | 0.1743 |
| Hu38-9 | 0.1976 |
| Hu38-10 | 0.1161 |
| Hu38-11 | 0.1683 |
| Hu38-12 | 0.1838 |
| Hu38-13 | 0.1866 |
| Hu38-14 | 0.1522 |
| Hu38-15 | 0.1493 |
| Hu38-16 | 0.1458 |
| Hu29-1 | 0.07111 |
| Hu29-2 | 0.06227 |
| Hu29-5 | 0.05531 |
| Hu29-6 | 0.08897 |
| Hu29-9 | 0.05608 |
| Hu29-10 | 0.08601 |
| Hu29-15 | 0.2158 |
| Hu29-16 | 0.2251 |
| Hu29-23 | 0.1184 |

The experimental results show that the anti-IL23 antibodies and the fusion proteins thereof of the present disclosure all have strong activity in inhibiting proliferation of BaF3-IL-23R cells.

### Test Example 4: IL-17 Secretion Experiment

The IL-17 secretion experiment was performed to detect the inhibition of the IL-23-induced T cell differentiation by the anti-IL23 antibodies and the fusion proteins thereof. The experimental procedures are as follows:
100 µL of 2 µg/mL anti-mouse CD3 antibody (BioLegend, 100238) and 2 µg/mL anti-mouse CD28 antibody (BioLegend, 102116) were added to each well of a 96-well plate (Corning, 3599). The plate was incubated at 37 °C for 1 h and then washed twice with PBS for later use. The spleens of mice were ground and centrifuged at 4 °C for 5 min, and lower-layer cells were collected, washed once with a washing solution (PBS + 2% FBS + 2 mM EDTA), and centrifuged. The supernatant was removed, the RBC Lysis Buffer (Invitrogen, 00-4333-57) was added, and the mixture was left to stand at room temperature for 5 min until the red blood cells were completely lysed. The lysate was centrifuged, and the cells were resuspended and counted. The cell suspension was sorted with the Mouse CD4 Cells Kit (Invitrogen, 11415D), and the isolated CD4⁺ T cells were resuspended in a medium of RPMI 1640 Medium (Gibco, 11875119) + 10% FBS (Gibco, 10099-141) and counted for later use. The cell suspension was plated on a coated 96-well plate. IL-23 (R&D Systems, 1290-IL-010) at a fixed concentration was mixed with the antibody or fusion protein diluted in a gradient, and the mixture was pre-incubated for 1 h and then added to the 96-well plate, and the resulting mixture was cultured in a cell incubator at 37 °C for 48 h. The 96-well plate was taken out and centrifuged at 1000 rpm for 3 min. The supernatant was collected and assayed for IL-17 content using the Mouse IL-17 DuoSet ELISA Kit (R&D Systems, DY421). The experimental results are shown in Table 17 below:

**Table 17. Experimental results of inhibiting IL-17 secretion**

| Sample name | IC₅₀ for inhibiting IL-17 secretion (nM) |
|---|---|
| Hu29-15 | 0.01954 |
| Hu29-19 | 0.01775 |
| Hu29-24 | 0.02890 |
| Hu29-19T | 0.01648 |
| Hu29-24T | 0.01278 |

The experimental results show that the anti-IL23 antibodies and the fusion proteins thereof of the present disclosure all have strong activity in inhibiting IL-17 secretion.

### Test Example 5: Experiment on Proliferation of B Cells

The experiment on proliferation of B cells was performed to determine whether the anti-IL23 antibody fusion proteins were able to inhibit the BAFF- and APRIL-induced proliferation of B cells. The experimental procedures are as follows:
The spleens of mice were ground and centrifuged at 4 °C for 5 min, and lower-layer cells were collected, washed once with a washing solution (PBS + 2% FBS + 2 mM EDTA), and centrifuged. The supernatant was removed, the RBC Lysis Buffer (Invitrogen, 00-4333-57) was added, and the mixture was left to stand at room temperature for 5 min until the red blood cells were completely lysed. The lysate was centrifuged, and the cells were resuspended and counted. The cell suspension was sorted using B Cell Isolation Kit (Miltenyi Biotec, 130-090-862), and the isolated B cells were resuspended in RPMI 1640 Medium (Gibco, 11875119) + 10% FBS (Gibco, 10099-141) + 50 µM 2-mercaptoethanol (Sigma-Aldrich, M6250) and counted. The cells were plated on a 96-well cell plate (Costar, 3903) for later use. The BAFF (R&D Systems, 7537-BF) or APRIL (R&D Systems, 5860-AP) protein was diluted to a fixed concentration, and the antibody or fusion protein diluted in a gradient was added. The mixture was mixed sufficiently, pre-incubated at 37 °C for 30 min, and then added to the 96-well cell plate. The cell plate was cultured in a cell incubator at 37 °C for 48 h. The cell culture plate was taken out, and the Celltiter Glo detection solution (Promega, G7573) was added at 50 µL/well. The plate was incubated at room temperature for 10 min. The bioluminescent signals were detected by a microplate reader, inhibition curves were fitted based on the detection results using software, and the IC₅₀ values were calculated. The experimental results are shown in Table 18 below:

**Table 18. Experimental results of inhibiting proliferation of B cells**

| Sample | Activity in inhibiting proliferation of B cells (IC₅₀, pM) | |
|---|---|---|
| | BAFF | APRIL |
| Hu29-19T | 5.176 | 8.111 |
| Hu29-24T | 4.783 | 4.802 |
| RCT-18 | 70.09 | 1987 |

The experimental results show that the inhibitory activity of the anti-IL23 antibody fusion proteins of the present disclosure for BAFF and APRIL is significantly stronger than that of the control RCT-18.

### Test Example 6: Affinity Assay

A certain amount of test sample was subjected to affinity capture by a biosensor chip Protein A (GE, 29127556), and then antigens at a series of concentration gradients were allowed to flow on the surface of the chip. The reaction signals were detected in real time by using Biacore (GE, 8K) to obtain association and dissociation curves. After the dissociation was completed for each cycle, the biochip was washed and regenerated with a 10 mM glycine-hydrochloric acid solution at pH 1.5 (GE, BR-1003-54). Fitting was performed based on the experimental data using BIAevaluation version 4.1 software with a 1:1 model, and affinity values were obtained. The relevant antigen proteins used in this assay are as follows: human IL-23 (Sino biological, CT048-H08H), human BAFF (Sino biological, 10056-HNCH), human APRIL (R&D Systems, 5860-AP-010/CF), cynomolgus monkey IL-23 (Aero Biosystems, ILB-CM52W8), cynomolgus monkey BAFF (Kactus, BAF-CM412), cynomolgus monkey APRIL (Kactus, APR-CM410B), mouse IL-23 (R&D Systems, 1887-ML/CF), mouse BAFF (Acro Biosystems, BAF-M521y), and mouse APRIL (R&D Systems, 7907-AP/CF). The results of the affinity assay are shown in Table 19 to Table 25 below:

**Table 19. Experimental results of affinity for the binding of anti-IL23 antibodies and fusion proteins thereof to human IL-23 p19**

| Sample | Human IL-23 p19 | | |
|---|---|---|---|
| | ka (1/Ms) | kd (1/s) | KD (M) |
| Hu29-19 | 2.71E+06 | 4.08E-05 | 1.51E-11 |
| Hu29-24 | 4.71E+06 | 7.03E-05 | 1.49E-11 |
| Hu29-19T | 2.37E+06 | 4.00E-05 | 1.69E-11 |
| Hu29-24T | 2.91E+06 | 4.22E-05 | 1.45E-11 |
| Risankizumab | 7.54E+05 | 4.91E-05 | 6.51E-11 |

**Table 20. Experimental results of affinity for the binding of anti-IL23 antibodies and fusion proteins thereof to cynomolgus monkey or mouse IL-23 p19**

| Sample | Cynomolgus monkey IL-23 p19 | | | Mouse IL-23 p19 |
|---|---|---|---|---|
| | ka (1/Ms) | kd (1/s) | KD (M) | |
| Hu29-19 | 2.71E+06 | 1.92E-04 | 7.08E-11 | No binding |
| Hu29-24 | 2.76E+06 | 2.36E-04 | 8.57E-11 | |
| Hu38-4 | 4.02E+05 | 3.09E-05 | 7.68E-11 | |
| Hu29-19T | 2.65E+06 | 1.90E-04 | 7.18E-11 | |
| Hu29-24T | 2.47E+06 | 1.50E-04 | 6.05E-11 | |

**Table 21. Experimental results of affinity for the binding of anti-IL23 antibody fusion proteins to human BAFF**

| Sample | Human BAFF | | |
|---|---|---|---|
| | ka (1/Ms) | kd (1/s) | KD (M) |
| Hu29-19T | 3.16E+06 | 7.08E-05 | 2.24E-11 |
| Hu29-24T | 2.51E+06 | 5.00E-05 | 2.00E-11 |
| RCT-18 | 9.64E+05 | 1.80E-04 | 1.86E-10 |

**Table 22. Experimental results of affinity for the binding of anti-IL23 antibody fusion proteins to cynomolgus monkey or mouse BAFF**

| Sample | Cynomolgus monkey BAFF | | | Mouse BAFF | | |
|---|---|---|---|---|---|---|
| | ka (1/Ms) | kd (1/s) | KD (M) | ka (1/Ms) | kd (1/s) | KD (M) |
| Hu29-19T | 6.78E+05 | 1.01E-04 | 1.49E-10 | 3.29E+06 | 7.79E-05 | 2.37E-11 |
| Hu29-24T | 5.83E+05 | 4.40E-05 | 7.55E-11 | 2.58E+06 | 7.14E-05 | 2.77E-11 |
| RCT-18 | 3.70E+05 | 9.54E-05 | 2.58E-10 | 7.40E+05 | 8.98E-05 | 1.21E-10 |

**Table 23. Experimental results of affinity for the binding of anti-IL23 antibody fusion proteins to human APRIL**

| Sample | Human APRIL | | |
|---|---|---|---|
| | ka (1/Ms) | kd (1/s) | KD (M) |
| Hu29-19T | 1.84E+08 | 1.89E-03 | 1.03E-11 |
| Hu29-24T | 2.15E+08 | 2.24E-03 | 1.04E-11 |
| RCT-18 | 1.72E+08 | 7.53E-03 | 4.39E-11 |

**Table 24. Experimental results of affinity for the binding of anti-IL23 antibody fusion proteins to cynomolgus monkey or mouse APRIL**

| Sample | Cynomolgus monkey APRIL | | | Mouse APRIL | | |
|---|---|---|---|---|---|---|
| | ka (1/Ms) | kd (1/s) | KD (M) | ka (1/Ms) | kd (1/s) | KD (M) |
| Hu29-19T | 1.55E+07 | 6.05E-05 | 3.90E-12 | 2.00E+07 | 3.60E-04 | 1.80E-11 |
| Hu29-24T | 7.18E+06 | 4.98E-04 | 6.94E-11 | 1.04E+07 | 2.07E-04 | 2.00E-11 |

**Table 25. Experimental results of affinity for the binding of anti-IL23 antibodies to human IL-23 p19**

| Antibody | Human IL-23 p19 | | |
|---|---|---|---|
| | ka (1/Ms) | kd (1/s) | KD (M) |
| Hu29-1 | 3.39E+06 | 1.33E-04 | 3.93E-11 |
| Hu29-2 | 3.51E+06 | 1.08E-04 | 3.08E-11 |
| Hu29-5 | 3.40E+06 | 1.25E-04 | 3.67E-11 |
| Hu29-6 | 3.86E+06 | 9.85E-05 | 2.55E-11 |
| Hu29-9 | 3.48E+06 | 1.06E-04 | 3.04E-11 |
| Hu29-10 | 3.65E+06 | 8.70E-05 | 2.39E-11 |
| Hu29-15 | 3.15E+06 | 7.02E-05 | 2.23E-11 |
| Hu29-16 | 4.36E+06 | 7.99E-05 | 1.83E-11 |
| Hu29-23 | 3.79E+06 | 8.57E-05 | 2.26E-11 |
| Ch29 | 4.05E+06 | 8.40E-05 | 2.07E-11 |
| Hu38-1 | 2.22E+05 | 4.56E-05 | 2.06E-10 |
| Hu38-2 | 3.37E+05 | 6.05E-05 | 1.80E-10 |
| Hu38-3 | 3.93E+05 | 4.44E-05 | 1.13E-10 |
| Hu38-4 | 3.53E+05 | 6.92E-05 | 1.96E-10 |
| Hu38-5 | 2.10E+05 | 5.82E-05 | 2.77E-10 |
| Hu38-6 | 2.73E+05 | 3.91E-05 | 1.43E-10 |
| Hu38-7 | 4.86E+05 | 7.41E-05 | 1.52E-10 |
| Hu38-8 | 2.82E+05 | 4.75E-05 | 1.68E-10 |
| Hu38-9 | 2.36E+05 | 6.68E-05 | 2.83E-10 |
| Hu38-10 | 3.58E+05 | 7.07E-05 | 1.97E-10 |
| Hu38-11 | 3.49E+05 | 6.15E-05 | 1.76E-10 |
| Hu38-12 | 3.21E+05 | 6.12E-05 | 1.91E-10 |
| Hu38-13 | 2.06E+05 | 5.56E-05 | 2.70E-10 |
| Hu38-14 | 3.12E+05 | 5.45E-05 | 1.75E-10 |
| Hu38-15 | 4.94E+05 | 8.30E-05 | 1.68E-10 |
| Hu38-16 | 3.41E+05 | 5.49E-05 | 1.61E-10 |
| Ch38 | 6.27E+05 | 5.65E-05 | 9.01E-11 |

The experimental results show that the anti-IL23 antibodies and the fusion proteins thereof constructed in the present disclosure can bind to human IL-23 p19 and cynomolgus monkey IL-23 p19 with high affinity, but do not bind to mouse IL-23 p19; in addition, the anti-IL23 antibody fusion proteins constructed in the present disclosure can bind to human BAFF, cynomolgus monkey BAFF, mouse BAFF, human APRIL, cynomolgus monkey APRIL, and mouse APRIL with high affinity.

### Test Example 7: Imiquimod-Induced Psoriasis Animal Model Experiment

The *in vivo* efficacy of anti-IL23 antibodies was assessed by an imiquimod cream (IMQ)-induced psoriasis animal model experiment.

SPF-grade female C57BL/6 hIL-23A/hIL12B transgenic mice (Biocytogen JiangSu Co., Ltd.) aged 8-9 weeks were randomly grouped with 5 mice in each group. The mice were depilated at the back prior to the start of the experiment. From day 0 to day 5, the mice in the sham surgery group were subjected to even application of 80 mg of vaseline (Unilever) on the skin of the back; mice in the animal model groups were subjected to even application of 80 mg of imiquimod cream (Best Medicine Store) on the skin of the back for 6 consecutive days. The test samples (risankizumab, Hu29-19, Hu29-24, and Hu38-4 all at a dose of 20 mpk) were intraperitoneally injected 1 h prior to application of imiquimod cream on day 0 and day 3. Risankizumab was used as a positive control and PBS was used as a negative control for a total of 2 administrations. The mice were scored daily for the severity of erythema and scaling of the back skin and the experiment ended on day 6.

The scoring criteria for the severity of erythema were as follows: no erythema, 0 points; reddish, 1 point; red but not dark, 2 points; deep red, 3 points; extremely red, 4 points. The scoring criteria for scaling were as follows: no scaling, 0 points; a small area of tiny pieces of skin, 1 point; a moderate area of relatively thick pieces of skin, 2 points; a relatively larger area of coarse and thickened pieces of skin, 3 points; a large area of large pieces of skin, 4 points.

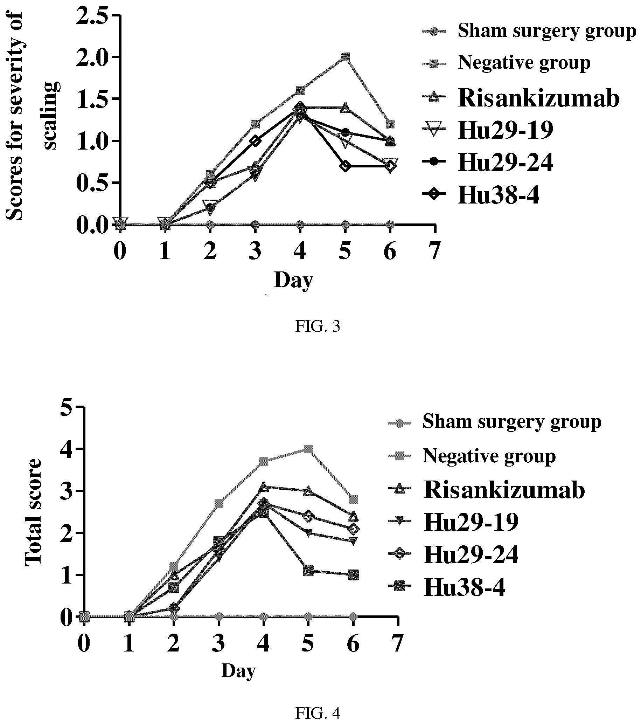

The experimental results are shown in FIGs. 2-4. The experimental results show that compared with the positive control risankizumab, the anti-IL23 antibodies constructed in the present disclosure have stronger *in vivo* efficacy, and the scores of the severity of erythema and the severity of skin scaling are lower than those of risankizumab.

### Test Example 8: Human IL-23-Induced Psoriasis Animal Model Experiment

The *in vivo* efficacy of anti-IL23 antibodies constructed in the present disclosure was assessed by a human IL-23-induced psoriasis animal model experiment. SPF-grade female C57BL/6J mice (Vital River Laboratory Animal Technology Co., Ltd.) aged 6-8 weeks were anesthetized, and then the right ear thickness and the body weight of the mice were measured. The mice were grouped according to the right ear thickness and the body weight, with 6 mice in each group. The day of grouping was recorded as day 0. From day 1, the mice were injected intradermally at the right ear with 1 µg of human IL-23 protein (R&D Systems, 1290-IL-500/CF) daily, for 7 days in total, and the mice in the normal group were injected with PBS. The test drugs were intraperitoneally injected on day 0 and day 3, and the body weight and the right ear thickness of the mice were measured and recorded on days 0, 2, 4, 6, and 8. The alleviation effect of the test drugs on the model was evaluated by the change in the right ear thickness. The experiment ended on day 8, and the ears with a diameter of 8 mm were collected and weighed.

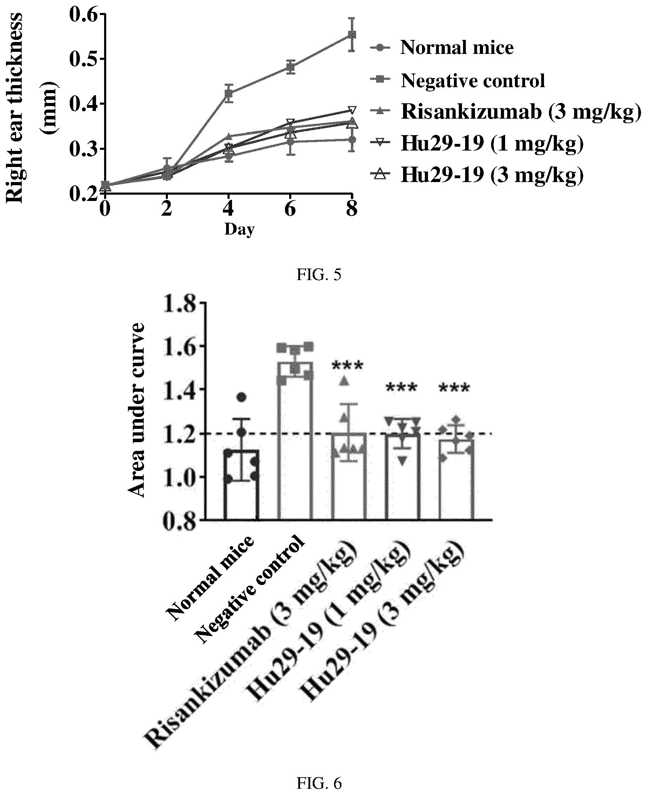

The experimental results are shown in FIGs. 5, 6 and 7. The experimental results show that the *in vivo* efficacy of the anti-IL23 antibody Hu29-19 constructed in the present disclosure in the human IL-23-induced psoriasis animal model is stronger than that of the control molecule risankizumab, wherein: when the doses were all 3 mpk, the ear thickness of the Hu29-19 group was lower than that of the control risankizumab; the statistical results of the area under the curve show that Hu29-19 exhibited a stronger effect than risankizumab at a dose of 3 mpk; statistical results of the mouse ear weight show that: the ear weight in the Hu29-19 1 mpk group was the same as that in the risankizumab 3 mpk group, and the ear weight in the Hu29-19 3 mpk group was lower than that in the risankizumab.

### Test example 9: Evaluation of In Vivo Activity in Inhibiting Cytokine Secretion

Two proteins, i.e., human IL-23 and human BAFF, were used to simultaneously stimulate the mice to induce the production of cytokines TNFα, IL-22, IgA, etc., in mice, and levels of these cytokines were determined to evaluate the *in vivo* activity of the antibodies or fusion proteins.

SPF-grade female C57BL/6 mice (Zhejiang Vital River Laboratory Animal Technology Co., Ltd.) aged 8 weeks were randomly grouped with 5 mice in each group. Two proteins, i.e., human IL-23 (Sino biological, CT048-H08H) (2 µg/mouse) and human BAFF (Sino biological, 10056-HNCH) (1 mg/kg), were mixed and injected intraperitoneally once daily for four days. The test samples (RCT-18 (4 mpk), risankizumab (8 mpk), Hu29-19T (8.8 mpk), and Hu29-24T (4.4 mpk)) were intraperitoneally injected 1 h prior to the injection of the proteins on day 1 and day 3. Plasma samples were collected on day 5 from mice in each group and tested for levels of TNFα, IL-22, and IgA. The source information of assay kits used in this test example is as follows: Mouse TNF-alpha Quantikine ELISA Kit (R&D Systems, MTA00B), Mouse/Rat IL-22 Quantikine ELISA Kit (R&D Systems, M2200), and Mouse IgA ELISA Kit (Abcam, ab157717). RCT-18 and risankizumab were used as positive controls, and PBS was used as a negative control. The drug molar concentrations of 4 mpk RCT-18, 8 mpk risankizumab and 8.8 mpk Hu29-19T were the same.

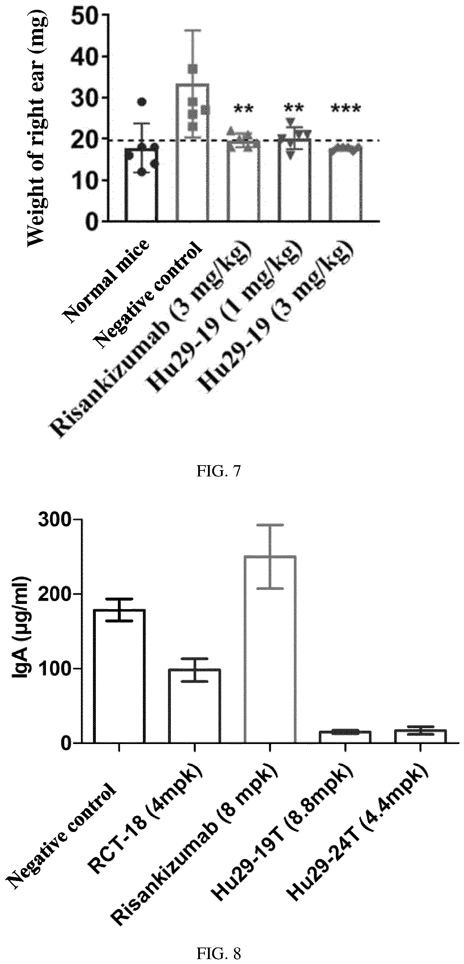

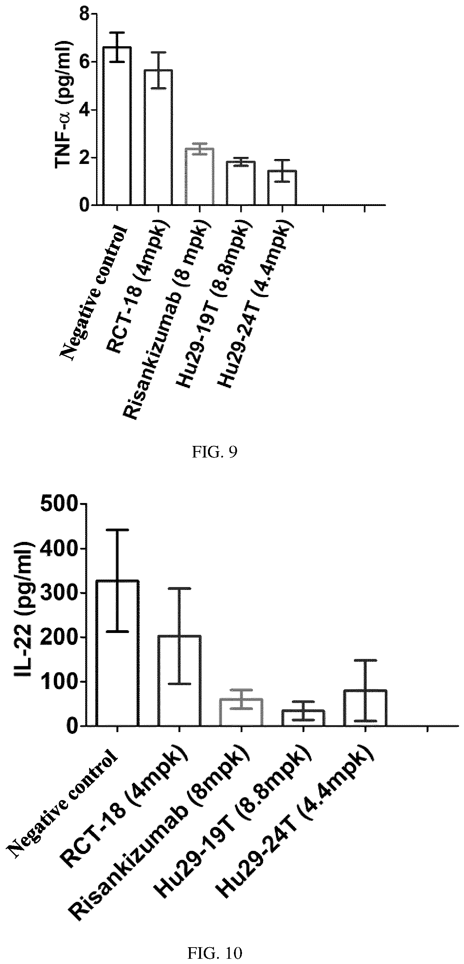

The results are shown in Tables 26, 27, and 28 and FIGs. 8, 9, and 10, wherein: the detection results for IgA show that the anti-IL23 antibody fusion proteins constructed in the present disclosure have remarkably stronger activity in inhibiting IgA secretion than the control molecule RCT-18, and risankizumab has no inhibition activity for IgA secretion; the detection results for TNFα show that under the doses of equimolar concentration, the anti-IL23 antibody fusion proteins constructed in the present disclosure have stronger activity in inhibiting secretion of TNFα than risankizumab, and RCT-18 does not show a remarkable inhibition effect; the detection results for IL-22 show that under the doses of equimolar concentration, the anti-IL23 antibody fusion proteins constructed in the present disclosure have stronger activity in inhibiting secretion of IL-22 than the controls risankizumab and RCT-18.

**Table 26. Detection results of IgA in mouse plasma (µg/mL)**

| Group | Negative control | RCT-18 (4mpk) | Risankizuma b (8 mpk) | Hu29-19T (8.8mpk) | Hu29-24T (4.4mpk) |
|---|---|---|---|---|---|
| Mean value of IgA (µg/mL) | 178.54 | 98.13 | 249.97 | 14.96 | 17.04 |

**Table 27. Detection results of TNFα in mouse plasma (µg/mL)**

| | | | | | |
|---|---|---|---|---|---|
| Mean value of TNFα (pg/mL) | 6.61 | 5.64 | 2.36 | 1.82 | 1.44 |

**Table 28. Detection results of IL-22 in mouse plasma (µg/mL)**

| Group | Negative control | RCT-18 (4mpk) | Risankizumab (8 mpk) | Hu29-19T (8.8mpk) | Hu29-24T (4.4mpk) |
|---|---|---|---|---|---|
| Mean value of IL-22 (pg/mL) | 326.86 | 202.48 | 60.00 | 34.38 | 79.71 |

### Test Example 10: In Vivo Efficacy in Systemic Lupus Erythematosus Animal Models

MRL/lpr mice spontaneously develop symptoms of an autoimmune disease similar to human systemic lupus erythematosus as the weeks of age increase. The *in vivo* efficacy of the fusion proteins was evaluated by this model.

Female MRL/lpr mice purchased from Shanghai Slac Laboratory Animal Co., Ltd. were housed at 5 mice/cage in an SPF-grade environment with a temperature of 20-25 °C and a humidity of 40-60 %. Mice were acclimatized in the laboratory environment for at least one week prior to the experiment. When the weeks of age of the mice reached 9-10 weeks and the values of urine protein and dsDNA IgG were significantly higher than those of normal control mice, administration via subcutaneous injection (RCT-18 (8 mpk), Hu29-19T (17.6 mpk), Hu29-19T (8.8 mpk), Hu29-24T (17.6 mpk), negative control (PBS)) was performed, wherein the molar concentrations of RCT-18 8 mpk, Hu29-19T 17.6 mpk, and Hu29-24T 17.6 mpk were the same. The administration was performed twice a week (1 time each on Monday and Thursday) for 8 consecutive weeks. Mice were observed regularly for hair and skin status, the skin damage severity of the mice was scored, and urine and plasma were collected to determine the urine protein content. After the experiment was completed, the kidneys of the mice were taken, fixed, and stained with H&E to observe the glomerular and tubular lesions and inflammatory cell infiltration of the kidney tissue of mice in each group and to score the degree of renal lesions. In this test example, the concentration of the urine protein was determined by using the Bradford Protein Assay Kit (purchased from Shanghai Beyotime Biological Tech. Co., Ltd.).

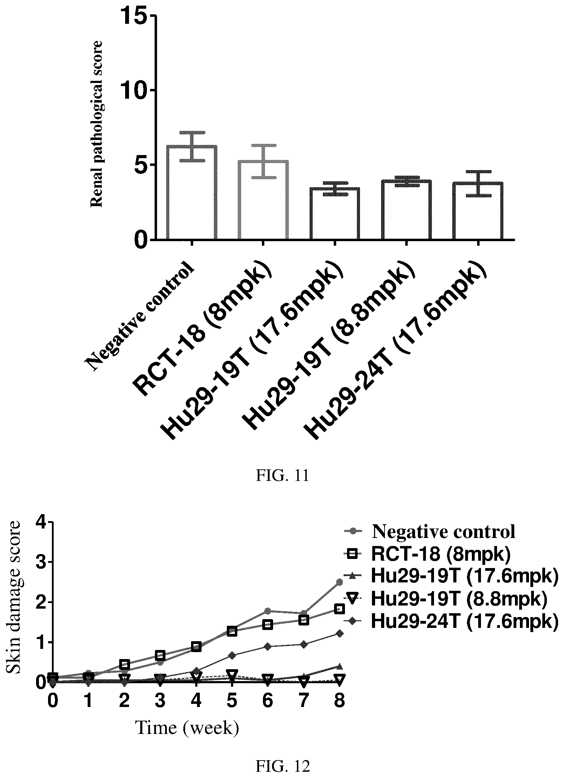

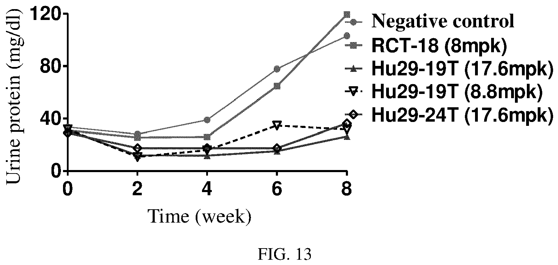

The experimental results are shown in Tables 29, 30, and 31 and FIGs. 11, 12, and 13. The experimental results show that RCT-18 did not lower the urine protein level in mice, Hu29-19T significantly decreased the urine protein level in mice at both doses, and the urine protein levels in mice of the Hu29-19T groups remained the same as or lower than the levels at the beginning of the experiment throughout the experiment and showed no tendency to increase at all. Hu29-24T also significantly reduced urine protein in mice. The skin injury scoring results of the mice show that the skin damage score of the mice in the Hu29-19T groups was always the lowest, the score of the Hu29-24T group was at a middle level, and RCT-18 did not show significant efficacy with regard to this index. The renal pathological scoring results of the mice show that the two dose groups of Hu29-19T had significant statistical difference compared with the negative group.

**Table 29. Renal pathological scores for mice**

| Group | Negative | RCT-18 (8mpk) | Hu29-19T (17.6mpk) | Hu29-19T (8.8mpk) | Hu29-24T (17.6mpk) |
|---|---|---|---|---|---|
| Mean value of renal pathological score | 6.22 | 5.22 | 3.40 | 3.89 | 3.75 |

**Table 30. Mean value of skin damage scores for mice**

| Number of weeks | Negative | RCT-18 (8mpk) | Hu29-19T (17.6mpk) | Hu29-19T (8.8mpk) | Hu29-24T (17.6mpk) |
|---|---|---|---|---|---|
| 0 | 0.11 | 0.11 | 0.00 | 0.00 | 0.00 |
| 1 | 0.22 | 0.11 | 0.05 | 0.06 | 0.00 |
| 2 | 0.28 | 0.44 | 0.05 | 0.06 | 0.00 |
| 3 | 0.50 | 0.67 | 0.05 | 0.06 | 0.11 |
| 4 | 0.83 | 0.89 | 0.05 | 0.11 | 0.28 |
| 5 | 1.33 | 1.28 | 0.10 | 0.17 | 0.67 |
| 6 | 1.78 | 1.44 | 0.05 | 0.06 | 0.89 |
| 7 | 1.72 | 1.56 | 0.15 | 0.00 | 0.94 |
| 8 | 2.50 | 1.83 | 0.40 | 0.06 | 1.22 |

**Table 31. Detection results of mouse urine protein (µg/dL)**

| Number of weeks | Negative | RCT-18 (8mpk) | Hu29-19T (17.6mpk) | Hu29-19T (8.8mpk) | Hu29-24T (17.6mpk) |
|---|---|---|---|---|---|
| 0 | 33.39 | 30.90 | 30.93 | 31.62 | 28.95 |
| 2 | 28.04 | 25.46 | 11.72 | 10.51 | 17.36 |
| 4 | 38.86 | 25.87 | 11.54 | 15.66 | 17.32 |
| 6 | 77.73 | 64.55 | 15.03 | 34.54 | 17.30 |
| 8 | 102.96 | 119.22 | 26.28 | 31.55 | 36.22 |

### Test Example 11: In Vivo Pharmacokinetic Experiment in Rats

An *in vivo* pharmacokinetic study was performed in SD rats. Male SD rats (Zhejiang Vital River Laboratory Animal Technology Co., Ltd.) were randomly grouped with 4 rats in each group, and the drugs were administered by intravenous injection. 0.2 mL of whole blood was collected before the administration and 5 min, 8 h, 24 h, 48 h, 84 h, 9 days, 10 days, 14 days, 21 days, and 28 days after the administration. Without anticoagulation, the collected blood was left to stand at 4 °C for 30 min and centrifuged at 1000 g for 15 min. The upper-layer serum was added to an EP tube and stored at -80 °C. The plasma concentration in serum was determined by ELISA, and pharmacokinetic parameters and *in vivo* half-life of test drugs were calculated by Winnolin software.

The experimental results of *in vivo* pharmacokinetics in rats are shown in Table 32 below. The experimental results show that the antibodies and the fusion proteins of the present disclosure have good stability with an *in vivo* half-life of more than 8 days.

**Table 32. Results of in vivo pharmacokinetic experiment in rats**

| Sample | Hu29-19T 13.2 mg/kg | | Hu29-24T 13.2 mg/kg | |
|---|---|---|---|---|
| Half-life | IL-23 end | TACI end | IL-23 end | TACI end |
| | 9.6±2.0 days | 8.8±1.9 days | 11.2±1.4 days | 10.5±1.4 days |

Although the foregoing invention has been described in detail by way of drawings and examples for purposes of clarity of understanding, the description and examples should not be construed as limiting the scope of the present disclosure. The disclosures of all patents and scientific literature cited herein are clearly incorporated by reference in their entirety.

## Claims

1. An anti-IL23 antibody fusion protein, comprising an anti-IL23 antibody and a TACI polypeptide, wherein the anti-IL23 antibody specifically binds to human IL23 p19 subunit;
preferably, the anti-IL23 antibody comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein
i) the HCDR1, the HCDR2, and the HCDR3 of the heavy chain variable region comprise amino acid sequences of a HCDR1, a HCDR2, and a HCDR3, respectively, in SEQ ID NO: 21, 22, 20, 19, 18, 17, or 1, and the LCDR1, the LCDR2, and the LCDR3 of the light chain variable region comprise amino acid sequences of a LCDR1, a LCDR2, and a LCDR3, respectively, in SEQ ID NO: 25, 26, 24, 23, or 2; or
ii) the HCDR1, the HCDR2, and the HCDR3 of the heavy chain variable region comprise amino acid sequences of a HCDR1, a HCDR2, and a HCDR3, respectively, in SEQ ID NO: 29, 30, 31, 32, or 3, and the LCDR1, the LCDR2, and the LCDR3 of the light chain variable region comprise amino acid sequences of a LCDR1, a LCDR2, and a LCDR3, respectively, in SEQ ID NO: 36, 35, 34, 33, or 4.

2. The anti-IL23 antibody fusion protein according to claim 1, wherein
i) in the heavy chain variable region, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
ii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16;
preferably,
i) in the heavy chain variable region, the HCDR1 comprises the amino acid sequence of SEQ ID NO: 27, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
ii) in the heavy chain variable region, the HCDR1 comprises the amino acid sequence of SEQ ID NO: 28, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
iii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37, and the LCDR3 comprises the amino acid sequence of SEQ ID NO: 16.

3. The anti-IL23 antibody fusion protein according to claim 1 or 2, wherein
i) the heavy chain variable region comprises any one of SEQ ID NO: 21, 22, 20, 19, 18, or 17, or an amino acid sequence having at least 90% sequence identity thereto, and the light chain variable region comprises any one of SEQ ID NO: 25, 26, 24, or 23, or an amino acid sequence having at least 90% sequence identity thereto; or
ii) the heavy chain variable region comprises any one of SEQ ID NO: 29, 30, 31, or 32, or an amino acid sequence having at least 90% sequence identity thereto, and the light chain variable region comprises any one of SEQ ID NO: 36, 35, 34, or 33, or an amino acid sequence having at least 90% sequence identity thereto; or
iii) the heavy chain variable region comprises SEQ ID NO: 1 or an amino acid sequence having at least 90% sequence identity thereto, and the light chain variable region comprises SEQ ID NO: 2 or an amino acid sequence having at least 90% sequence identity thereto; or
iv) the heavy chain variable region comprises SEQ ID NO: 3 or an amino acid sequence having at least 90% sequence identity thereto, and the light chain variable region comprises SEQ ID NO: 4 or an amino acid sequence having at least 90% sequence identity thereto;
preferably, i) the heavy chain variable region comprises the amino acid sequence of SEQ ID NO: 21, and the light chain variable region comprises the amino acid sequence of SEQ ID NO: 25; or
ii) the heavy chain variable region comprises the amino acid sequence of SEQ ID NO: 22, and the light chain variable region comprises the amino acid sequence of SEQ ID NO: 26; or
iii) the heavy chain variable region comprises the amino acid sequence of SEQ ID NO: 29, and the light chain variable region comprises the amino acid sequence of SEQ ID NO: 36.

4. The anti-IL23 antibody fusion protein according to any one of claims 1-3, wherein the anti-IL23 antibody further comprises antibody heavy and light chain constant regions;
preferably, the heavy chain constant region is selected from the group consisting of human IgG1, IgG2, IgG3, and IgG4 constant regions, and the light chain constant region is selected from the group consisting of human antibody κ chain and λ chain constant regions;
more preferably, the heavy chain constant region comprises the amino acid sequence of SEQ ID NO: 38, and the light chain constant region comprises the amino acid sequence of SEQ ID NO: 39.

5. The anti-IL23 antibody fusion protein according to any one of claims 1-4, wherein the anti-IL23 antibody comprises a heavy chain and a light chain, wherein
i) the heavy chain of the anti-IL23 antibody comprises SEQ ID NO: 40 or an amino acid sequence having at least 90% sequence identity thereto, and the light chain of the anti-IL23 antibody comprises SEQ ID NO: 41 or an amino acid sequence having at least 90% sequence identity thereto; or
ii) the heavy chain of the anti-IL23 antibody comprises SEQ ID NO: 42 or an amino acid sequence having at least 90% sequence identity thereto, and the light chain of the anti-IL23 antibody comprises SEQ ID NO: 43 or an amino acid sequence having at least 90% sequence identity thereto; or
iii) the heavy chain of the anti-IL23 antibody comprises SEQ ID NO: 44 or an amino acid sequence having at least 90% sequence identity thereto, and the light chain of the anti-IL23 antibody comprises SEQ ID NO: 45 or an amino acid sequence having at least 90% sequence identity thereto;
preferably,
i) the heavy chain of the anti-IL23 antibody comprises the amino acid sequence of SEQ ID NO: 40, and the light chain of the anti-IL23 antibody comprises the amino acid sequence of SEQ ID NO: 41; or
ii) the heavy chain of the anti-IL23 antibody comprises the amino acid sequence of SEQ ID NO: 42, and the light chain of the anti-IL23 antibody comprises the amino acid sequence of SEQ ID NO: 43; or
iii) the heavy chain of the anti-IL23 antibody comprises the amino acid sequence of SEQ ID NO: 44, and the light chain of the anti-IL23 antibody comprises the amino acid sequence of SEQ ID NO: 45.

6. The anti-IL23 antibody fusion protein according to any one of claims 1-5, wherein the TACI polypeptide is a polypeptide comprising amino acid residues at positions 48-85 of SEQ ID NO: 58 or a variant thereof, wherein the variant has an amino acid replacement at one or more positions selected from the group consisting of positions 49, 52, 53, 57, 65, 82, and 83, wherein the position for the amino acid replacement is an amino acid residue position numbered in natural order relative to the sequence SEQ ID NO: 58; preferably, the variant has one or more amino acid replacements selected from the group consisting of 49T or 49R, 52S, 53E or 53Q, 57E, 65T or 65A, 82A or 82R, and 83Y, wherein the position for the amino acid replacement is an amino acid residue position numbered in natural order relative to the sequence SEQ ID NO: 58.

7. The anti-IL23 antibody fusion protein according to any one of claims 1-6, wherein the amino acid sequence of the TACI polypeptide is set forth in any one of SEQ ID NOs: 51-83;
preferably, the amino acid sequence of the TACI polypeptide is set forth in SEQ ID NO: 83.

8. The anti-IL23 antibody fusion protein according to any one of claims 1-7, comprising:
a first chain: [a TACI polypeptide 1]-[a linker 1]-[the heavy chain of the anti-IL23 antibody]-[a linker 2]-[a TACI polypeptide 2], and
a second chain: the light chain of the anti-IL23 antibody, wherein
the TACI polypeptide 1 and the TACI polypeptide 2 are identical or different and are independently selected from any TACI polypeptide defined in claims 6 and 7, and the linker 1 and the linker 2 are identical or different;
preferably, the linker is a linker selected from the group consisting of (GₓS)_{y}, wherein x is selected from the group consisting of integers from 1 to 5 and y is selected from the group consisting of integers from 0 to 6; more preferably, the linker is a linker set forth in SEQ ID NO: 84 or 85.

9. The anti-IL23 antibody fusion protein according to any one of claims 1-8, wherein the anti-IL23 antibody fusion protein has a first chain comprising the amino acid sequence of SEQ ID NO: 46 and a second chain comprising the amino acid sequence of SEQ ID NO: 41; or
the anti-IL23 antibody fusion protein has a first chain comprising the amino acid sequence of SEQ ID NO: 47 and a second chain comprising the amino acid sequence of SEQ ID NO: 43.

10. An anti-IL23 antibody, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein
i) the HCDR1, the HCDR2, and the HCDR3 of the heavy chain variable region comprise amino acid sequences of a HCDR1, a HCDR2, and a HCDR3, respectively, in SEQ ID NO: 21, 22, 20, 19, 18, 17, or 1, and the LCDR1, the LCDR2, and the LCDR3 of the light chain variable region comprise amino acid sequences of a LCDR1, a LCDR2, and a LCDR3, respectively, in SEQ ID NO: 25, 26, 24, 23, or 2; or
ii) the HCDR1, the HCDR2, and the HCDR3 of the heavy chain variable region comprise amino acid sequences of a HCDR1, a HCDR2, and a HCDR3, respectively, in SEQ ID NO: 29, 30, 31, 32, or 3, and the LCDR1, the LCDR2, and the LCDR3 of the light chain variable region comprise amino acid sequences of a LCDR1, a LCDR2, and a LCDR3, respectively, in SEQ ID NO: 36, 35, 34, 33, or 4.

11. The anti-IL23 antibody according to claim 10, wherein
i) in the heavy chain variable region, the HCDR1 comprises the amino acid sequence of SEQ ID NO: 27, 28, or 5, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
ii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37 or 15, and the LCDR3 comprises the amino acid sequence of SEQ ID NO: 16;
preferably,
i) in the heavy chain variable region, the HCDR1 comprises the amino acid sequence of SEQ ID NO: 27, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
ii) in the heavy chain variable region, the HCDR1 comprises the amino acid sequence of SEQ ID NO: 28, the HCDR2 comprises the amino acid sequence of SEQ ID NO: 6, and the HCDR3 comprises the amino acid sequence of SEQ ID NO: 7; in the light chain variable region, the LCDR1 comprises the amino acid sequence of SEQ ID NO: 8, the LCDR2 comprises the amino acid sequence of SEQ ID NO: 9, and the LCDR3 comprises the amino acid sequence of SEQ ID NO: 10; or
iii) in the heavy chain variable region, the HCDR1 comprises the amino acid sequence of SEQ ID NO: 11, the HCDR2 comprises the amino acid sequence of SEQ ID NO: 12, and the HCDR3 comprises the amino acid sequence of SEQ ID NO: 13; in the light chain variable region, the LCDR1 comprises the amino acid sequence of SEQ ID NO: 14, the LCDR2 comprises the amino acid sequence of SEQ ID NO: 37, and the LCDR3 comprises the amino acid sequence of SEQ ID NO: 16.

12. The anti-IL23 antibody according to claim 10 or 11, wherein
i) the heavy chain variable region comprises any one of SEQ ID NO: 21, 22, 20, 19, 18, or 17, or an amino acid sequence having at least 90% sequence identity thereto, and the light chain variable region comprises any one of SEQ ID NO: 25, 26, 24, or 23, or an amino acid sequence having at least 90% sequence identity thereto; or
ii) the heavy chain variable region comprises any one of SEQ ID NO: 29, 30, 31, or 32, or an amino acid sequence having at least 90% sequence identity thereto, and the light chain variable region comprises any one of SEQ ID NO: 36, 35, 34, or 33, or an amino acid sequence having at least 90% sequence identity thereto; or
iii) the heavy chain variable region comprises SEQ ID NO: 1 or an amino acid sequence having at least 90% sequence identity thereto, and the light chain variable region comprises SEQ ID NO: 2 or an amino acid sequence having at least 90% sequence identity thereto; or
iv) the heavy chain variable region comprises SEQ ID NO: 3 or an amino acid sequence having at least 90% sequence identity thereto, and the light chain variable region comprises SEQ ID NO: 4 or an amino acid sequence having at least 90% sequence identity thereto;
preferably, i) the heavy chain variable region comprises the amino acid sequence of SEQ ID NO: 21, and the light chain variable region comprises the amino acid sequence of SEQ ID NO: 25; or
ii) the heavy chain variable region comprises the amino acid sequence of SEQ ID NO: 22, and the light chain variable region comprises the amino acid sequence of SEQ ID NO: 26; or
iii) the heavy chain variable region comprises the amino acid sequence of SEQ ID NO: 29, and the light chain variable region comprises the amino acid sequence of SEQ ID NO: 36.

13. The anti-IL23 antibody according to any one of claims 10-12, wherein the anti-IL23 antibody further comprises antibody heavy and light chain constant regions;
preferably, the heavy chain constant region is selected from the group consisting of human IgG1, IgG2, IgG3, and IgG4 constant regions, and the light chain constant region is selected from the group consisting of human antibody κ chain and λ chain constant regions;
more preferably, the heavy chain constant region comprises the amino acid sequence of SEQ ID NO: 38, and the light chain constant region comprises the amino acid sequence of SEQ ID NO: 39.

14. The anti-IL23 antibody according to any one of claims 10-13, wherein the anti-IL23 antibody comprises a heavy chain and a light chain, wherein
i) the heavy chain of the anti-IL23 antibody comprises SEQ ID NO: 40 or an amino acid sequence having at least 90% sequence identity thereto, and the light chain of the anti-IL23 antibody comprises SEQ ID NO: 41 or an amino acid sequence having at least 90% sequence identity thereto; or
ii) the heavy chain of the anti-IL23 antibody comprises SEQ ID NO: 42 or an amino acid sequence having at least 90% sequence identity thereto, and the light chain of the anti-IL23 antibody comprises SEQ ID NO: 43 or an amino acid sequence having at least 90% sequence identity thereto; or
iii) the heavy chain of the anti-IL23 antibody comprises SEQ ID NO: 44 or an amino acid sequence having at least 90% sequence identity thereto, and the light chain of the anti-IL23 antibody comprises SEQ ID NO: 45 or an amino acid sequence having at least 90% sequence identity thereto;
preferably,
i) the heavy chain of the anti-IL23 antibody comprises the amino acid sequence of SEQ ID NO: 40, and the light chain of the anti-IL23 antibody comprises the amino acid sequence of SEQ ID NO: 41; or
ii) the heavy chain of the anti-IL23 antibody comprises the amino acid sequence of SEQ ID NO: 42, and the light chain of the anti-IL23 antibody comprises the amino acid sequence of SEQ ID NO: 43; or
iii) the heavy chain of the anti-IL23 antibody comprises the amino acid sequence of SEQ ID NO: 44, and the light chain of the anti-IL23 antibody comprises the amino acid sequence of SEQ ID NO: 45.

15. The anti-IL23 antibody fusion protein according to any one of claims 1-9 or the anti-IL23 antibody according to any one of claims 10-14, having one or more of the following properties:
A. specifically binding to human IL-23 p19 and cynomolgus monkey IL-23 p19, but not specifically binding to mouse IL-23 p19; preferably, binding to human IL-23 p19 with a KD value of less than 6.00E-11 M and/or binding to cynomolgus monkey IL-23 p19 with a KD value of less than 9.00E-11 M, the KD values being measured by BIACORE^{®} surface plasmon resonance assay;
B. having the activity of blocking IL-23/IL-23R binding; preferably, blocking human IL-23/IL-23R binding with an IC₅₀ value of less than 0.60 nM, the IC₅₀ value being measured by Elisa;
C. having the activity of blocking BAFF/BAFF-R binding; preferably, blocking BAFF/BAFF-R binding with an IC₅₀ value of less than 7.00 nM, the IC₅₀ value being measured by Elisa;
D. having the activity of blocking BAFF/BCMA binding; preferably, blocking BAFF/BCMA binding with an IC₅₀ value of less than 4.50 nM, the IC₅₀ value being measured by Elisa;
E. having the activity of blocking BAFF/TACI binding; preferably, blocking BAFF/TACI binding with an IC₅₀ value of less than 6.00 nM, the IC₅₀ value being measured by Elisa;
F. having the activity of blocking APRIL/BCMA binding; preferably, blocking APRIL/BCMA binding with an IC₅₀ value of less than 1.00 nM, the IC₅₀ value being measured by Elisa;
G. having the activity of blocking APRIL/TACI binding; preferably, blocking APRIL/TACI binding with an IC₅₀ value of less than 3.00 nM, the IC₅₀ value being measured by Elisa;
H. having the activity of inhibiting IL-17 secretion; preferably, inhibiting IL-17 secretion with an IC₅₀ value of less than 0.03 nM, the IC₅₀ value being measured by Elisa;
I. having the activity of inhibiting proliferation of BaF3-IL-23R cells; preferably, inhibiting proliferation of BaF3-IL-23R cells with an IC₅₀ value of less than 0.5 nM, the IC₅₀ value being measured by PerkinElmer;
J. having the activity of inhibiting secretion of cytokines TNFα, IL-22, and IgA; or
K. having the activity of inhibiting proliferation of B cells.

16. A pharmaceutical composition, comprising the anti-IL23 antibody fusion protein according to any one of claims 1-9 and 15 or the anti-IL23 antibody according to any one of claims 10-15, and
one or more pharmaceutically acceptable carriers, diluents, or excipients.

17. A nucleic acid molecule encoding the anti-IL23 antibody fusion protein according to any one of claims 1-9 and 15 or the anti-IL23 antibody according to any one of claims 10-15.

18. A host cell, comprising the nucleic acid molecule according to claim 17.

19. A method for treating or ameliorating a B cell disorder or an autoimmune disease, comprising the step of administering to a subject in need thereof the anti-IL23 antibody fusion protein according to any one of claims 1-9 and 15, the anti-IL23 antibody according to any one of claims 10-15, or the pharmaceutical composition according to claim 16, wherein
preferably, the B cell disorder or the autoimmune disease is a disease or condition associated with IL23 expression;
more preferably, the autoimmune disease is selected from the group consisting of systemic lupus erythematosus, myasthenia gravis, multiple sclerosis, insulin-dependent diabetes mellitus, Crohn's disease, rheumatoid arthritis, polyarticular juvenile rheumatoid arthritis, and psoriatic arthritis; the B cell disorder is selected from the group consisting of tumors, chronic leukocytic leukemia, multiple myeloma, non-Hodgkin's lymphoma, post-transplant lymphoproliferative disorder, and light chain gammopathy;
most preferably, the autoimmune disease is systemic lupus erythematosus.
